(19) Europäisches Patentamt
European Patent Office
Office européen des brevets

(11) EP 4 349 869 A1

(12) EUROPEAN PATENT APPLICATION
published in accordance with Art. 153(4) EPC

(43) Date of publication:
**10.04.2024 Bulletin 2024/15**

(21) Application number: **22811709.9**

(22) Date of filing: **30.05.2022**

(51) International Patent Classification (IPC):
***C07K 19/00*** (2006.01) ***C07K 14/005*** (2006.01)
***A61K 39/12*** (2006.01) ***A61K 9/51*** (2006.01)
***A61P 31/14*** (2006.01)

(52) Cooperative Patent Classification (CPC):
**A61K 9/51; A61K 39/12; A61P 31/14; C07K 14/005; C07K 19/00**

(86) International application number:
**PCT/KR2022/007697**

(87) International publication number:
**WO 2022/250518 (01.12.2022 Gazette 2022/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2021 US 202163194310 P**

(71) Applicant: **LEMONEX INC.**
**Seoul 06683 (KR)**

(72) Inventor: **WON, Cheol Hee**
**Seoul 08741 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

Remarks:
The complete document including Reference Table(s) and the Sequence Listing(s) can be downloaded from the EPO website

# (54) VACCINE FOR PREVENTION OR TREATMENT OF VIRAL INFECTION

(57) The present invention relates to a nucleic acid molecule of RBD-(L)n-X sequence (wherein, RBD is a sequence of at least a partial region including the receptor-binding domain of the spike protein, L is a linker sequence, n is 0 or 1, and X is the nucleotide sequence of SEQ ID NO: 1), and a virus vaccine composition including the nucleic acid molecule. Preferably, the molecule may be used in a vaccine composition against various viral infections.

[FIG. 19]

Balb/c (female)
0 1 2 3 4 5 6 Weeks
Buffer
Seq 3
1st
2nd
Serum analysis

**
**
Inhibition (%)
120
100
80
60
40
20
0
0
14
21
35
Days after initial immunization

EP 4 349 869 A1

## Description

[Technical Field]

The present invention relates to a vaccine composition including a carrier for carrying a nucleic acid with uses for prevention and treatment of a viral infection.

[Background Art]

Recent viral infections including COVID-19 progress to an epidemic stage around the world within a short period of time after occurrence, thus rapid development of vaccines is required. However, conventional development of a vaccine has a problem of taking a long period of time from a preclinical phase to the final use due to many stages including basic research, clinical trials, approval, manufacturing and sales.

The mRNA vaccine is a vaccine having a mechanism that induces the establishment of an acquired immune system against viruses by injecting mRNA containing genetic information of a virus into the human body, causing viral proteins such as spike proteins to be synthesized in the body, and thereby inducing the human immune system to detect the synthesized proteins and create an antibody against the virus. Recently, using this mRNA vaccine technology, there are attempts to develop a replaceable basic technology by analyzing the viral gene sequence, and then changing only specific antigens or genetic information of a genetic material in the vaccine platform, that is, existing vaccine. Based on the mRNA vaccine platform technology in which toxicity profile analysis and manufacturing procedures are established, Moderna of the United States shortened the period of time from virus sequence selection after pathogen identification to the start of phase 1 clinical trials from previous 20 months to 3 months.

However, there are problems in that mRNA vaccines are unstable due to the nature of their molecular structure thus to be easily decomposed, the delivery efficiency to target cells is not high, or the amino acid encoded by the mRNA sequence does not have high antigenicity or immunogenicity. Therefore, researches on the better mRNA vaccine technology is urgently needed.

[Summary of Invention]

[Problems to be Solved by Invention]

It is an object of the present invention to provide a nucleic acid molecule encoding a viral receptor-binding domain which forms a stable structure.

Another object of the present invention is to provide a carrier for carrying the nucleic acid molecule.

In addition, another object of the present invention is to provide a vaccine for prevention or treatment of a viral infection.

[Means for Solving Problems]

1. A nucleic acid molecule of the following sequence:

RBD-(L)n-X

(wherein, RBD is a sequence of at least a partial region including a receptor-binding domain in a spike protein, L is a linker sequence, n is 0 or 1, and X is the nucleotide sequence of SEQ ID NO: 1).

2. The nucleic acid molecule according to the above 1, wherein the RBD is a virus-derived sequence forms a triplet by the receptor-binding domain thereof.

3. The nucleic acid molecule according to the above 1, wherein the RBD has a length of 100 nt to 5,000 nt.

4. The nucleic acid molecule according to the above 1, wherein the RBD is the nucleotide sequence of SEQ ID NO: 2.

5. The nucleic acid molecule according to the above 1, wherein L is the nucleotide sequence of SEQ ID NO: 3 or SEQ ID NO: 4.

6. The nucleic acid molecule according to the above 1, wherein the RBD is a virus-derived sequence selected from the group consisting of *herpesviridae, orthomyxoviridae, rhabdoviridae, paramyxoviri dae, papilomaviridae, adenoviridae, parvoviridae, astrovirid ae, reoviridae, bunyaviridae, arteriviridae, caliciviridae, hepeviridae, bornaviridae, arenaviridae, togaviridae, filoviridae, retroviridae, flaviv iridae* and *coronaviridae.*

7. The nucleic acid molecule according to the above 1, wherein the RBD is a virus-derived sequence selected from the group consisting of *Colacovirus, Decacovirus, Duvinacovirus, Luchavirus, Minacovirus, Minunacovirus, Myota covirus, Nyctacovirus, Pedacovirus, Rhinacovirus, Setracovir us, Soracovirus, Tegacovirus, Embecovirus, Hibecovirus, Merb ecovirus, Nobecovirus, Sarbecovirus, Brangacovirus, Cegacovi rus, Igacovirus, Andecovirus, Buldecovirus* and *Herdecovirus.*

8. A virus vaccine composition including the nucleic acid molecule according to any one of the above 1 to 7.

9. The virus vaccine composition according to the above 8, wherein the nucleic acid molecule is carried on a carrier selected from the group consisting of a viral carrier, virus-like particles (VLPs), positively charged polymer, liposome, lipid nanoparticles, gold, semiconductor quantum dots, carbon nanotube and porous silica particles.

10. The virus vaccine composition according to the above 8, wherein the nucleic acid molecule is carried on porous silica particles.

11. The virus vaccine composition according to the above 10, wherein the porous silica particles have an average pore diameter of 5 nm to 100 nm.

12. The virus vaccine composition according to the above 10, wherein the porous silica particles are positively charged inside the pores.

13. The virus vaccine composition according to the above 10, wherein the porous silica particles are biodegradable particles.

14. The virus vaccine composition according to the above 10, wherein a weight ratio of the nucleic acid molecule to the porous silica particles is 1:1 to 30.

[Advantageous effects]

The present invention provides a nucleic acid molecule including a nucleotide sequence encoding a sequence of at least a partial region which includes the receptor-binding domain of a spike protein, a linker sequence, and the nucleotide sequence of SEQ ID NO: 1. Preferably, the nucleic acid molecule is carried on the carrier, thus to be utilized as a vaccine composition with a high prevention rate against the viral infection.

[Brief Description of Drawings]

FIG. 1 is micrographs of porous silica particles according to an embodiment of the present invention.

FIG. 2 is micrographs of porous silica particles according to an embodiment of the present invention.

FIG. 3 is micrographs of small pore particles obtained in a manufacturing process of the porous silica particles according to an embodiment of the present invention.

FIG. 4 is micrographs of the small pore particles according to an embodiment of the present invention.

FIG. 5 is micrographs of the porous silica particles for each pore diameter according to an embodiment of the present invention. A degradable carrier (DDV) is the particles according to an embodiment, wherein the number in parenthesis means a diameter of the particle and the number of subscripts means a pore diameter. For example, DDV $200_{10}$ refers to a particle having a particle diameter (that is, particle size) of 200 nm and a pore diameter of 10 nm according to an embodiment.

FIG. 6 is micrographs to identify biodegradability of the porous silica particles according to an embodiment of the present invention.

FIG. 7 a view illustrating a tube having a cylindrical permeable membrane according to one illustrative example.

FIG. 8 shows results of decreasing absorbance of the porous silica particles over time according to an embodiment of the present invention.

FIG. 9 shows results of decreasing absorbance of the porous silica particles for each particle size over time according to an embodiment of the present invention.

FIG. 10 shows results of decreasing absorbance of the porous silica particles for each pore diameter over time according to an embodiment of the present invention.

FIG. 11 shows results of decreasing absorbance of the porous silica particles for each pH of the environment over time according to an embodiment of the present invention.

FIG. 12 shows results of decreasing absorbance of the porous silica particles over time according to an embodiment of the present invention.

FIG. 13 shows results of confirming the weight ratio of porous silica particles according to an embodiment of the present invention to RBD-Ln-X nucleic acid; and the loading efficiency of the porous silica particles according to the nucleic acid sequence.

FIG. 14 shows results of confirming the loading efficiency of the porous silica particles when Covid mRNA was carried on the porous silica particles according to an embodiment of the present invention, and the nucleic acid sequence, storage temperature, and sucrose content of a buffer were varied.

FIG. 15 shows results of confirming the RBD translation efficiency when RBD-L n-X nucleic acid was carried on the porous silica particles according to an embodiment of the present invention and delivered *in vitro.*

FIG. 16 shows results of confirming the amount of RBD-specific IgG when RBD-$L_n$-X nucleic acid was carried on the porous silica particles according to an embodiment of the present invention and delivered *in vivo.*

FIG. 17 shows results of confirming the amount of antibody produced according to the dose of Seq. 3 when Seq. 3 was carried on the porous silica particles according to an embodiment of the present invention and delivered *in vivo.*

FIG. 18 shows results of confirming the amount of binding antibody produced over time when Seq. 3 was carried on the porous silica particles according to an embodiment of the present invention and delivered *in vivo.*

FIG. 19 shows results of confirming the amount of neutralizing antibody produced over time when Seq. 3 was carried on the porous silica particles according to an embodiment of the present invention and delivered *in vivo.*

FIG. 20 shows results of confirming the expression location and duration of luciferase when Luc mRNA was carried on the porous silica particles according to an embodiment of the present invention and injected submuscularly *in vivo.*

FIG. 21 shows results of confirming the expression location and duration of luciferase when Luc mRNA was carried on the porous silica particles according to an embodiment of the present invention and injected intratumorally *in vivo.*

FIG. 22 shows results of confirming the expression duration and expression intensity of luciferase when Luc mRNA was carried on the porous silica particles according to an embodiment of the present invention and injected submuscularly *in vivo.*

FIG. 23 shows results of confirming the expression location and expression level of luciferase over time after injection when Luc mRNA was carried on the porous silica particles according to an embodiment of the invention and stored at 4 °C or 25 °C for 7 days and 14 days, respectively.

FIG. 24 shows results of confirming the expression level of luciferase according to storage time and storage temperature after injection by carrying Luc mRNA on the porous silica particles according to an embodiment of the invention.

[Mode for Carrying out Invention]

Hereinafter, the present invention will be described in detail. Unless otherwise specifically defined, all terms in the present specification would have the same meanings as general meanings of the corresponding terms understood by persons having common knowledge to which the present invention pertains ("those skilled in the art"), and if the general meanings conflict with the meanings of the terms used herein, the meanings used in the present specification take precedence.

The present invention relates to a nucleic acid molecule of the following sequence:

RBD-(L)n-X

(wherein, RBD is a sequence of at least a partial region including a receptor-binding domain in a spike protein, L is a linker sequence, n is 0 or 1, and X is the nucleotide sequence of SEQ ID NO: 1).

In the present invention, the RBD is included in the scope of the present invention without limitation as long as it includes a nucleotide sequence encoding a virus-derived domain that binds to a receptor present in a host cell. Preferably, the RBD forms a triplet by the receptor-binding domain thereof on the surface of the virus, and more specifically, is the nucleotide sequence of SEQ ID NO: 2, but it is not limited thereto.

The RBD may be included in the scope of the present invention without limitation on the length of the sequence, as long as it can express effects as a vaccine if it includes at least a portion of the receptor-binding domain region in the spike protein. For example, the RBD may further include a nucleotide sequence at 5'- or 3'-terminus of its sequence, which encodes an amino acid at N-terminus or C-terminus of the receptor-binding domain region of the spike protein. More specifically, the RBD sequence may have a length of 100 nt to 5,000 nt, 200 nt to 4,000 nt, 300 nt to 3,000 nt, 400 nt to 2,000 nt, 500 nt to 1000 nt, etc., but it is not limited thereto.

In addition, the RBD may be included in the scope of the present invention regardless of the type of the virus, as long as it is a domain constituting the spike protein located on the surface of the virus. For example, the RBD may be a virus-derived sequence belonging to the virus family selected from the group consisting of *herpesviridae, orthomyxoviridae, rhabdoviridae, paramyxoviri dae, papilomaviridae, adenoviridae, parvoviridae, astrovirid ae, reoviridae, bunyaviridae, arteriviridae, caliciviridae, hepeviridae, bornaviridae, arenaviridae, togaviridae, filoviridae, retroviridae, flaviv iridae* and *coronaviridae,* and more specifically, may be a virus-derived sequence selected from the group consisting of *Colacovirus, Decacovirus, Duvinacovirus, Luchavirus, Minacovirus, Minunacovirus, Myota covirus, Nyctacovirus, Pedacovirus, Rhinacovirus, Setracovir us, Soracovirus, Tegacovirus, Embecovirus, Hibecovirus, Merb ecovirus, Nobecovirus, Sarbecovirus, Brangacovirus, Cegacovi rus, Igacovirus, Andecovirus, Buldecovirus* and *Herdecovirus,* but it is not limited thereto.

If the RBD and the nucleotide sequence of SEQ ID NO: 1 are linked, they may be included in the scope of the present invention without limitation. For example, the nucleotide sequence encoding RBD and the nucleotide sequence of SEQ ID NO: 1 may be directly linked, or the RBD may be linked to the nucleotide sequence of SEQ ID NO: 1 through a linker. As the linker, those known in the art may be used without limitation, and for example, saturated alkyl chain (C3 to C18), trizole linker, 4-methyl-6,7,8,9,10,10a-hexahydro-5H-3λ2-cycloocta[d]pyridazine linker, etc. may be used. In terms of minimizing side reactions and side effects in the body, the saturated alkyl chain is preferably used. As the saturated alkyl chain, C3 to C18, C3 to C15, C3 to C12, C3 to C10, C4 to C15, C4 to C12, C4 to C10, C4 to C8, C5 to C15, C5 to C12, C5 to C10, C3 to C8, C3 to C6, or C4 to C6 may be used. Specifically, an amino acid bound by the nucleotide sequences of SEQ ID NOs: 3 to 5, and encoded by the nucleotide sequence may be used as the linker. Preferably, when an amino acid bound by the nucleotide sequence of SEQ ID NO: 3 or SEQ ID NO: 4 and encoded by the nucleotide sequence is used as the linker, the efficiency as a vaccine may be further improved, but it is not limited thereto.

In the present invention, the nucleotide sequence of SEQ ID NO: 1 may be linked to the RBD to increase RBD translation efficiency and/or vaccine production efficiency. The improvement in the efficiency may be achieved by inducing the amino acid sequence encoded from the nucleic acid to form a triplet, and may be achieved by increasing the binding stability of the viral receptor-binding domain that has formed the triplet, but it is not limited thereto.

In the present invention, the nucleic acid may further include a signal peptide sequence. The signal peptide sequence may be a nucleotide sequence inserted into 5'-terminus or 3'-terminus region of the nucleic acid, and the length thereof is not limited. The signal peptide sequence may allow a nucleic acid having such effects to be inserted therein depending on the purpose without limitation. For example, the signal peptide sequence may be intended to improve the stability of the nucleic acid and/or the amino acid encoded therefrom, to increase the translation efficiency of the amino acid, or may be used for secreting the synthesized amino acid out of the cell. The signal peptide sequence may have a length of, for example, 1 to 200 nt, 10 to 150 nt, or 20 to 100 nt, etc. More specifically, the sequence of SEQ ID NO: 6 may be added to the 5'-terminus of the nucleic acid, but it is not limited thereto.

In the present invention, the nucleic acid may further include a termination codon at the 3'-terminus. For example, TGA, TAG, and TAA sequences may be added to the 3'-terminus of SEQ ID NO: 1, and the sequences may be repeated one to several times.

In the present invention, the base constituting the nucleic acid molecule may be DNA or RNA, and even if the nucleotide sequence includes a base that has undergone chemical modification, it is included in the scope of the present invention without limitation. The base that has undergone chemical modification may be, for example, pseudouridine, N1-methylpseudouridine, N1-ethylpseudouridine, 2-thiouridine, 4'-thiouridine, 5-methylcytosine, 2-thio-1-methyl-1-deaza-pseudouridine, 2-thio-1-methyl-pseudouridine, 2-thio-5-aza-uridine, 2-thio-dihydrospseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine or 2'-O-methyl uridine. The translation efficiency of the nucleic acid may be increased by the chemically modified base, but it is not limited thereto.

The present invention relates to a virus vaccine composition including the above-described nucleic acid molecule.

When the virus vaccine composition of the present invention is introduced into target cells, it may synthesize the amino acid sequence encoded by RBD-(L)n-X sequence included in the composition. When recognized by immune cells, the amino acid sequence may have high antigenicity or immunogenicity to play a role of a vaccine against an infection caused by the virus including the RBD. As described above, the virus vaccine composition of the present invention may exhibit higher translation efficiency and/or vaccine production efficiency than the case where the RBD exists alone.

The virus vaccine composition of the present invention may have a form in which the nucleic acid molecule is carried on a carrier. As long as the carrier is capable of delivering the carried vaccine composition to the target cells, it may be included in the scope of the present invention regardless of the types thereof. For example, the carrier may include a viral carrier, virus-like particles (VLPs), positively charged polymer, liposome, lipid nanoparticles, gold, semiconductor quantum dots, carbon nanotube, or porous silica particles, and preferably, is carried on the porous silica particles, but it is not limited thereto.

In order to be carried on the carrier, the nucleic acid molecule may further have a functional group coupled thereto, which is capable of binding to the carrier at the 5'-terminus and/or 3'-terminus. In this regard, functional groups capable of coupling to each other or compounds having the same in the fields of biology and chemistry may be applied to the nucleic acid molecule without limitation, and the nucleic acid molecule may be carried on the carrier through a coupling reaction between these functional groups.

In the present invention, the porous silica particles of the present invention are particles based on silica ($SiO_2$) material and have a nano-scale particle size, and correspond to a carrier capable of carrying the nucleic acid on the surface thereof and/or inside the pores. The particle may have a plurality of pores, wherein the pores may extend from the surface to the inside of the particle and may be connected to each other. In the present invention, when the nucleic acid is carried in the porous silica particles and delivered to cells, delivery efficiency into the cells may be improved compared to the case where the nucleic acid is not carried on the silica particles, and the nucleic acid carried therein may be gradually released, such that the retention time in blood may be increased. In addition, silica particles may exhibit higher delivery effects than other carriers described above.

In the present invention, the average particle diameter of the porous silica particles is included in the scope of the present invention without limitation as long as they can carry the nucleic acid, and may be selected by those skilled in the art depending on conditions such as the type or amount of the nucleic acid to be carried. The particles may have an average particle diameter of, for example, 50 nm to 1,000 nm. The average particle diameter may be selected by those skilled in the art depending on conditions such as the weight of nucleic acid carried therein. The lower limit of the average particle diameter may be, for example, 50 nm, 60 nm, 70 nm, 80 nm or 90 nm, and the upper limit thereof may be, for example, 1,000 nm, 900 nm, 800 nm, 700 nm, or 600 nm, but it is not limited thereto.

By having an average pore diameter within the above range, the porous silica particles may sufficiently support or carry the nucleic acid molecules inside the pores and deliver to the cells. The pore diameter may be selected in a smaller range depending on the size of the average particle diameter described above. The larger the diameter of the particle, the easier it is to carry nucleic acid, and the greater the weight of nucleic acid it may carry, but the rate of release of nucleic acid may be proportional to that. The pore diameter may be selected without limitation according to the purpose of those skilled in the art, and the lower limit may be, for example, 5 nm, 7 nm, 10 nm, 12 nm, 15 nm, 17 nm, or 20 nm, and the upper limit may be, for example, 100 nm, 90 nm, 80 nm, 70 nm, 60 nm or 50 nm, but it is not limited thereto.

The porous silica particles are positively charged inside the pores, and may have a zeta potential of 5 to 80 mV. Within the above range, the zeta potential may be, for example, 5 mV to 80 mV, 5 mV to 70 mV, 5 mV to 60 mV, 5 mV to 55 mV, 10 mV to 80 mV, 10 mV to 70 mV, 10 mV to 60 mV, 10 mV to 55 mV, 20 mV to 80 mV, 20 mV to 70 mV, 20 mV to 60 mV, 20 mV to 55 mV, 30 mV to 80 mV, 30 mV to 70 mV, 30 mV to 60 mV, 30 mV to 55 mV, 40 mV to 80 mV, 40 mV to 70 mV, 40 mV to 60 mV, 40 mV to 55 mV, 50 mV to 80 mV, 50 mV to 70 mV, 50 mV to 60 mV, or 50 mV to 55 mV, etc., but it is not limited thereto.

When the charged particles are absorbed into a target cell (e.g., through a process such as endocytosis), the particles entering the cell may have a strong positive charge due to low pH in endosome, which may induce osmosis due to diffusion of water passing a membrane of the endosome, thereby leading to formation of vacuoles.

When the particles have a strong positive charge, the cell membrane is wider than when it wraps around the particles, whereby extracellular fluid or foreign substances such as various proteins contained therein other than the particles may be introduced into the target cells together. In such a case, unexpected effects may occur due to inflow of foreign materials or, compared to the case where there is no inflow of foreign materials, the particles may relatively less inflow. Therefore, it may be difficult to generate drug effects by sufficient delivery of the particles. On the other hand, if the positive charge of the particles is weak, the loading efficiency may be decreased. However, the present invention may prevent the problems described above by optimizing the charge of nucleic acid-carrying particles.

The loading ratio of nucleic acid molecule to the particles may be, for example, a weight ratio of the porous silica particles to the nucleic acid molecule of 1: 1 to 30. When a content ratio is within the above range, it is possible to prevent the generation of empty porous silica particles in which the nucleic acid molecule is not carried, while sufficiently carrying the nucleic acid molecule, whereby the particles having a strong positive charge are prevented from being delivered to the cells. Within the above range, the loading ratio may be, for example, 1:1 to 30, 1: 3 to 20, 1: 3 to 15, 1: 3 to 12, 1: 3 to 10, 1: 5 to 20, 1: 5 to 15, 1: 5 to 10, etc., but it is not limited thereto.

Specifically, in order to administer the composition using the carrier of the present invention to a subject, the porous silica particles carrying the nucleic acid molecules may be dispersed in a dispersion medium, which may be obtained by adding and stirring the porous silica particles and the nucleic acid molecules in a dispersion medium. In this regard, if an amount of particles relative to the nucleic acid molecules is too large, empty particles not sufficiently carrying the nucleic acid molecules may occur, and if the amount of particles relative to the nucleic acid molecules is too small, residual nucleic acid molecules not carried by the particles may be generated.

In one embodiment of the present invention, the porous silica particles may have the following specifications. For example, the pore size may range from 7 to 30 nm, specifically, 12 to 18 nm, and more specifically 14 to 16 nm. The particle diameter may range from 50 to 1,000 nm, specifically 200 to 500 nm, and more specifically 250 to 350 nm. The surface area may range from 200 to 700 $m^2/g$, and specifically, 360 to 480 $m^2/g$. The zeta potential may be 5 mV or more, specifically 20 to 70 mV, and more specifically 40 to 60 mV in the state before carrying of the nucleic acid molecule. The particles may carry the nucleic acid molecules to reach a weight ratio of 1:1 to 30, and specifically 1: 5 to 25, 1: 5 to 20, 1: 10 to 25, 1: 10 to 20.

The porous silica particles of the present invention are biodegradable particles, which carry the nucleic acid molecules and are biodegradable in the body to release the nucleic acid molecules when administered into the body. However, the porous silica particles of the present invention are slowly degraded in the body so that the carried nucleic acid molecules can be released in a sustained manner. For example, t at which an absorbance ratio in Equation 1 below is 1/2 may be 20 or more:

[Equation 1]

$$A_t/A_0$$

(wherein $A_0$ is absorbance of the porous silica particles measured by putting 5 mL of suspension containing 1 mg/mL of the porous silica particles into a cylindrical permeable membrane having pores with a pore diameter of 50 kDa, 15 mL of the same solvent as the suspension comes into contact with an outside of the permeable membrane, and the inside/outside of the permeable membrane are horizontally stirred at 60 rpm and 37 °C, pH of the suspension is 7.4, and $A_t$ indicates absorbance of the porous silica particles measured after lapse of "t" hours since $A_o$ was measured).

The above Equation 1 means what a rate the porous silica particles are degraded in an environment similar to the body, and absorbance $A_0$ and $A_t$ in the above Equation 1 may be measured after placing porous silica particles and a suspension in a cylindrical permeable membrane and also placing the same suspension outside the permeable membrane.

The porous silica particles of the present invention are biodegradable, and may be slowly degraded in the suspension. The diameter of 50 kDa corresponds to about 5 nm, which allows the biodegraded porous silica particles to pass through a permeable membrane having a diameter of 50 kDa, and a cylindrical permeable membrane is under horizontal agitation at 60 rpm to evenly blend the suspension, such that the degraded porous silica particles can come out of the permeable membrane.

The absorbance in the above Equation 1 may be measured, for example, under an environment in which the suspension outside the permeable membrane is replaced with a new suspension. The suspension may be continuously replaced, or replaced every period wherein the period is periodic or irregular. For example, the suspension may be replaced in a 1-hour interval, 2-hour interval, 3-hour interval, 6-hour interval, 12-hour interval, 24-hour interval, 2-day interval, 3-day interval, 4-day interval, or 7-day interval, etc., within a range of 1 hour to 1 week, but it is not limited thereto.

The meaning in which the absorbance ratio becomes 1/2 refers to that the absorbance is half of the initial absorbance after t hours, briefly, that approximately half of the porous silica particles are degraded.

The suspension may be a buffer solution, for example, at least one selected from the group consisting of phosphate buffered saline (PBS) and simulated body fluid (SBF), and more specifically, PBS.

"t" in the above Equation 1, at which the absorbance ratio becomes 1/2, may be 20 or more, for example, t may range from 24 to 120. That is, within the above range, t may range from 20 to 96, 24 to 96, 24 to 72, 30 to 70, 40 to 70, or 50 to 65, etc., but it is not limited thereto.

With regard to the porous silica particles, t at which the absorbance ratio in the above Equation 1 becomes 1/5 may range from 70 to 140. For example, t may range from 80 to 140, 80 to 120, 80 to 110, 70 to 140, 70 to 120, or 70 to 110, etc. within the above range, but it is not limited thereto.

With regard to the porous silica particles, t at which the absorbance ratio in the above Equation 1 becomes 1/20 may range from 130 to 220. For example, t may range from 130 to 200, 140 to 200, 140 to 180, or 150 to 180, etc. within the above range, but it is not limited thereto.

With regard to the porous silica particles, t at which the absorbance ratio in the above Equation 1 becomes 0.01 or less may be 250 or more, 300 or more, 350 or more, 400 or more, 500 or more, or 1,000 or more, etc. and the upper limit may be 2,000, but it is not limited thereto.

With regard to the porous silica particles, the absorbance ratio and t in the above Equation 1 have high positive correlation. For example, Pearson correlation coefficient may be 0.8 or more, and for example, 0.9 or more, or 0.95 or more.

"t" in the above Equation 1 means how fast the porous silica particles are degraded under the environment similar to the body. That is, t may be regulated by adjusting, for example, a surface area, a particle size, a pore diameter, substituents on the surface of the porous silica particles and/or the inside the pores, compactness of the surface and the like. For example, the surface area of the particle may be increased to reduce t, or the surface area may be decreased to increase t. The surface area may be regulated by adjusting the particle size and the pore diameter of the particles. Further, if direct exposure of the porous silica particles to the environment (such as solvents) is reduced by placing substituents on the surface of the particles and/or the inside the pores, t may be increased. Further, when the porous silica particles support or carry the nucleic acid molecule, and when increasing affinity between the nucleic acid molecule and the porous silica particles, direct exposure of the porous silica particles to the environment may be reduced, thereby increasing t. In addition, t may be increased by preparing the particles with more compact surface during manufacturing. As described above, various examples of adjusting t in the above Equation 1 have been described, but it is not limited thereto.

The porous silica particles of the present invention may have a spherical shape, but it is not limited thereto.

The porous silica particles of the present invention may have a BET surface area of, for example, 200 to 700

$m^2$/g, but it is not limited thereto. For example, the BET surface area may range from 200 $m^2$/g to 700 $m^2$/g, 220 $m^2$/g to 680 $m^2$/g, 220 $m^2$/g to 620 $m^2$/g, 280 $m^2$/g to 680 $m^2$/g, 280 $m^2$/g to 580 $m^2$/g, 280 $m^2$/g to 520 $m^2$/g, 280 $m^2$/g to 480 $m^2$/g, 320 $m^2$/g to 620 $m^2$/g, 320 $m^2$/g to 580 $m^2$/g, 320 $m^2$/g to 520 $m^2$/g, 320 $m^2$/g to 480 $m^2$/g, 320 $m^2$/g to 450 $m^2$/g, 320 $m^2$/g to 420 $m^2$/g, 360 $m^2$/g to 480 $m^2$/g, or 360 $m^2$/g to 420 $m^2$/g, etc. within the above range, but it is not limited thereto.

Porous silica nanoparticles of the present invention may have a volume per gram, for example, 0.7 to 2.2 mL. For example, the volume may range from 0.7 to 2.0 mL, 0.8 to 2.2 mL, 0.8 to 2.0 mL, 0.9 to 2.0 mL, or 1.0 to 2.0 mL, etc. within the above range, but it is not limited thereto.

The porous silica particles may include pores of small pore particles having an average pore diameter of less than 5 nm expanded to an average diameter of 7 to 30 nm. As a result, the pore diameter is large so that large nucleic acid molecule can be carried inside the pores, and the particle diameter itself is not large compared to the pore diameter, so that it is easy to deliver and absorb into the cells.

The porous silica particles of the present invention may be positively charged at an outer surface thereof and/or inside the pores. For example, both the surface of the particles and the inside the pores may be positively charged, or only the surface of the particles or the inside the pores may be positively charged. The charging may be performed, for example, by the presence of a cationic substituent.

The cationic substituent may include, for example, an amino group or any other nitrogen-containing group as a basic group. Further, the anionic substituent may include, for example, carboxy group (-COOH), sulfonic acid group (-$SO_3$H), or thiol group (-SH), etc. as an acidic group, but it is not limited thereto.

The positively charging may be performed by reacting the porous silica particles with alkoxysilane having a basic group such as a nitrogen-containing group, for example, an amino group or an aminoalkyl group. Specifically, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silane, or 3-(2-aminoethylamino)propyldimethoxymethylsilane, etc. may be used. The positively charging may be performed by reacting the porous silica particles with a polymer having a basic group such as a nitrogen-containing group. For example, polyetherimide (PEI), polydially dimethyl ammonium chloride (PDADMAC), poly-acrylamide (CPAM), polyamine epichlorohydrin (PAE), or poly-aluminium chloride (PAC), etc. may be used as the polymer. The polymer may be selected regardless of the molecular weight, charge, etc. thereof, as long as it can modify the outer surface of the porous silica particles and/or the inside the pores, but it is not limited thereto.

When positively charging the porous silica particles by reacting them with the polymer, these particles may be charged with a higher charge than the case of reacting them with a compound containing an amine group. Thus, when the nucleic acid molecules are carried on the porous silica particles, it is possible to exhibit higher loading efficiency due to electrical bonding between the nucleic acid and amine groups. When interaction of the porous silica particles with the release environment of the nucleic acid molecules is regulated by adjusting the substituents through charging described above, the degradation rate of the nanoparticles themselves may be regulated to control a release rate of the nucleic acid molecules. Further, the nucleic acid molecules may be diffused and released from the nanoparticles. In this regard, adjusting the substituents may regulate a binding force of the nucleic acid molecule to the nanoparticles, thereby controlling release of the nucleic acid molecules.

Further, the porous silica particles may include substituents present therein for the purposes of: carrying the nucleic acid molecule on the surface of the particles and/or the inside the pores; delivery of the nucleic acid molecule into the target cells; carrying other substances for other purposes; or otherwise, binding of additional substituents. In addition, the porous silica particles may further include antibodies, ligands, cell permeable peptides, or aptamers, etc., which are bound thereto.

The above-described substituents, charge, binders, etc. on the surface of the particles and/or the inside the pores may be added by, for example, surface modification. Surface modification may be performed, for example, by reacting a compound or polymer having a substituent to be introduced with the particles. The compound may be, for example, alkoxysilane having C1 to C10 alkoxy group, and the polymer may be, for example, polyetherimide (PEI), polydially dimethyl ammonium chloride (PDADMAC), poly-acrylamide (CPAM), polyamine epichlorohydrin (PAE), or poly-aluminium chloride (PAC), etc., but it is not limited thereto. The alkoxysilane has one or more alkoxy groups, for example, 1 to 3 alkoxy groups. Further, there may be a substituent to be introduced into a site where the alkoxy group is not bound, or a substituent substituted with the same.

When modifying the surface of the porous silica particles and/or the inside the pores with the polymer, for example, when using a polymer having a molecular weight of 100,000 or more, a space for carrying the nucleic acids inside the pores may not be secured, thereby causing a decrease in the loading efficiency. The molecular weight of the available polymer may be, for example, 100,000 or less, 90,000 or less, 70,000 or less, 60,000 or less, 50,000 or less, or 40,000 or less, etc. The lower limit thereof may be, for example, 500 or more, 1,000 or more, 2,000 or more, 3,000 or more, 4,000 or more, or 5,000 or more, etc. The surface modification may be performed by methods known to those skilled in the art without limitation. For example, modification may be performed by putting the porous silica particles into a solution in which the compound or polymer to be modified is dissolved/dispersed, followed by drying the same, but it is not limited thereto.

For example, the porous silica particles may be manufactured, for example, through small pore particle preparation and pore expansion processes, and if necessary, may be manufactured further through calcination, surface modification process or the like. If both the calcination and the surface modification processes have been implemented, the particles may be surface-modified after calcination.

The vaccine composition of the present invention may maintain effects thereof for a predetermined period of time even when stored at a higher temperature than the conventional vaccine compositions including mRNA as a major component. For example, even when stored at -70 °C to 25 °C for 2 weeks, the amount of nucleic acids carried on the porous silica particles may not be decreased, and the nucleic acids carried on the porous silica particles may not be decomposed or have no modification in the sequence thereof, thus to also synthesize a normal protein. Preferably, it is advantageous to store the vaccine composition at a low temperature, but the storage may also be possible at a temperature of 25 °C, 20 °C, 15 °C, 10 °C, 4 °C or 0 °C.

Due to the high translation efficiency and/or neutralizing antibody production ability of the nucleic acid; or the high delivery efficiency of the carrier, the vaccine composition of the present invention may induce a high immune response even if the composition does not include an adjuvant. However, the vaccine composition may further include an adjuvant to induce a higher immune response. The adjuvant may be selected by those skilled in the art without limitation as long as it is intended to enhance the effects of the vaccine composition. Examples of the adjuvant may include at least one selected from the group consisting of Alum (Aluminium salts), IL-12, GM-CSF (Granulocyte-macrophage colony-stimulating factor), squalene, MF59, AS03, AS04, poly (I:C), MPL (Monophosphoryl Lipid A), GLA, flagellin, Imiquimod, R848, CpG ODN, CpG DNA, saponins (QS-21), C-type lectin ligands (TDB), α-galactosylceramide, muramyl dipeptide, lipopolysaccharide (LPS), Kuil A, AS01 (liposome mixed with monophosphoryl lipid A and saponin QS-21), IC31 (oligo nucleotide and cationic peptide), CFA01 (cationic liposome), and GLA-SE (oil-in-water emulsion of MPL and glucopyranosyl lipid), but they are not limited thereto.

The vaccine composition of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be additionally added to formulate the vaccine composition into injectable formulations, pills, capsules, granules or tablets such as aqueous solutions, suspensions, emulsions and the like.

The vaccine composition of the present invention is applicable in a form of any formulation, and may be prepared in oral or parenteral formulations. The pharmaceutical formulations of the present invention may include forms suitable for oral, rectal, nasal, topical (including the cheek and sublingual), subcutaneous, vaginal or parenteral (submuscular, subcutaneous) administration.

The porous silica particles of the vaccine composition bind to cells at the introduced local site when administered by submuscular injection, tumor injection, etc. and deliver the nucleic acids carried therein, thereby allowing the vaccine composition to be specifically delivered to a specific tissue, but it is not limited thereto. For example, in the case of intravenous injection, the nucleic acid carried therein may be released throughout the body along the blood vessels.

The vaccine composition of the present invention is administered in a pharmaceutically effective amount. Effective dose levels may be determined depending on types or severity of disease of the patient, activity of drug, sensitivity to drug, administration time, administration route and rate of release, duration of treatment, factors including concurrent medications, and other factors well known in the medical field. The vaccine composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the vaccine composition in an amount that can achieve maximum effects with a minimum amount without side effects, which may be easily determined by those skilled in the art.

An administration amount, that is, dosage of the vaccine composition of the present invention may vary widely depending on the weight, age, sex, health conditions or diet of a patient, administration time, administration method, excretion rate and severity of the disease, and the appropriate dosage depends on, for example, an amount of drug accumulated in the patient's body and/or specific efficacy of the carrier of the present invention used. For example, the amount may be from 0.01 μg to 1 g per kg of body weight. Further, the vaccine composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

Hereinafter, the present invention will be described in detail with reference to the following examples.

## Example 1. Preparation of porous silica particles (DegradaBALL)

### 1. Preparation of porous silica particles

#### (1) Preparation of porous silica particles

##### 1) Preparation of small pore particles

960 mL of distilled water (DW) and 810 mL of MeOH were put into a 2 L round bottom flask. 7.88 g of CTAB was added to the flask, followed by rapid addition of 4.52 mL of 1M NaOH under stirring. After adding a homogeneous mixture while stirring for 10 minutes, 2.6 mL of TMOS was further added. After stirring for 6 hours to mix uniformly, the reaction solution was aged for 24 hours.

Then, the reaction solution was centrifuged at 8,000 rpm and 25 °C for 10 minutes to remove the supernatant, and washed five times with ethanol and distilled water alternately while centrifuging at 8,000 rpm and 25 °C for 10 minutes.

Thereafter, the resultant product was dried in an oven at 70 °C to harvest 1.5 g of powdery microporous silica particles (pore average diameter of 2 nm, and particle size of 200 nm).

##### 2) Pore expansion

1.5 g of microporous silica particle powder was added to 10 mL of ethanol and subjected to ultrasonic dispersion, and 10 mL of water and 10 mL of TMB (trimethyl benzene) were further added, followed by ultrasonic dispersion.

Thereafter, the dispersion was placed in an autoclave and reacted at 160 °C for 48 hours.

The reaction was initiated at 25 °C and performed while raising the temperature at a rate of 10 °C/min, then slowly cooled in an autoclave at a rate of 1 to 10 °C/min.

The cooled reaction solution was centrifuged at 8,000 rpm and 25 °C for 10 minutes to remove the supernatant, and washed five times with ethanol and distilled water alternately while centrifuging at 8,000 rpm and 25 °C for 10 minutes.

Then, the product was dried in an oven at 70 °C to harvest powdery porous silica particles (pore diameter of 10 to 15 nm, and particle size of 200 nm).

##### 3) Calcination

The porous silica particles prepared in 2) above were put in a glass vial, heated at 550 °C for 5 hours, and cooled slowly to room temperature after completing the reaction to prepare particles.

#### (2) Preparation of porous silica particles

Porous silica particles were prepared by the same method as Example 1-1-(1) above, except that the reaction conditions at the time of pore expansion were changed to 140 °C and 72 hours.

#### (3) Preparation of porous silica particles (10 L scale)

Porous silica particles were prepared by the same method as Example 1-1-(1), except that a 5 times larger container was used and each material was used in a 5 times capacity.

#### (4) Preparation of porous silica particles (particle size of 300 nm)

Porous silica particles were prepared by the same method as Example 1-1-(1), except that 920 mL of distilled water and 850 mL of methanol were used to prepare the small pore particles.

#### (5) Preparation of porous silica particles (particle size of 500 nm)

Porous silica particles were prepared by the same method as Example 1-1-(1), except that 800 mL of distilled water, 1,010 mL of methanol, and 10.6 g of CTAB were used to prepare the small pore particles.

**(6) Preparation of porous silica particles (particle size of 1,000 nm)**

Porous silica particles were prepared by the same method as Example 1-1-(1), except that 620 mL of distilled water, 1,380 mL of methanol, and 7.88 g of CTAB were used to prepare the small pore particles.

**(7) Preparation of porous silica particles (pore diameter of 4 nm)**

Porous silica particles were prepared by the same method as Example 1-1-(1), except that 2.5 mL of TMB was used for pore expansion.

**(8) Preparation of porous silica particles (pore diameter of 7 nm)**

Porous silica particles were prepared by the same method as Example 1-1-(1), except that 4.5 mL of TMB was used for pore expansion.

**(9) Preparation of porous silica particles (pore diameter of 17 nm)**

Porous silica particles were prepared by the same method as Example 1-1-(1), except that 11 mL of TMB was used for pore expansion.

**(10) Preparation of porous silica particles (pore diameter of 23 nm)**

Porous silica particles were prepared by the same method as Example 1-1-(1), except that 12.5 mL of TMB was used for pore expansion.

**(11) Preparation of porous silica particles**

Porous silica particles were prepared by the same method as Example 1-1-(1), except that 900 mL of distilled water, 850 mL of methanol and 8 g of CTAB were used in preparation of small pore particles.

**2. Surface modification of porous silica particles**

(1) The porous silica particles of Example 1-1-(1) were reacted with (3-aminopropyl)triethoxysilane (APTES) to be positively charged.

Specifically, 100 mg of porous silica particles were dispersed in a 10 mL toluene in a 100 mL round bottom flask with a bath sonicator. Then, 1 mL of APTES was added and stirred at 400 rpm and 130 °C for 12 hours.

After the reaction, the product was slowly cooled to room temperature and centrifuged at 8,000 rpm for 10 minutes to remove the supernatant, and washed five times with ethanol and distilled water alternately while centrifuging at 8,000 rpm and 25 °C for 10 minutes.

Thereafter, the product was dried in an oven at 70 °C to harvest powdery porous silica particles having an amino group on the surface thereof and inside the pores.

(2) The product of Example 1-1-(11) was subjected to surface modification by the same method described above except that 1.8 mL of APTES was used, thereby obtaining powdery porous silica particles having amino groups on the surface of the particles and inside the pores.

**3. Identification of particle formation and pore expansion**

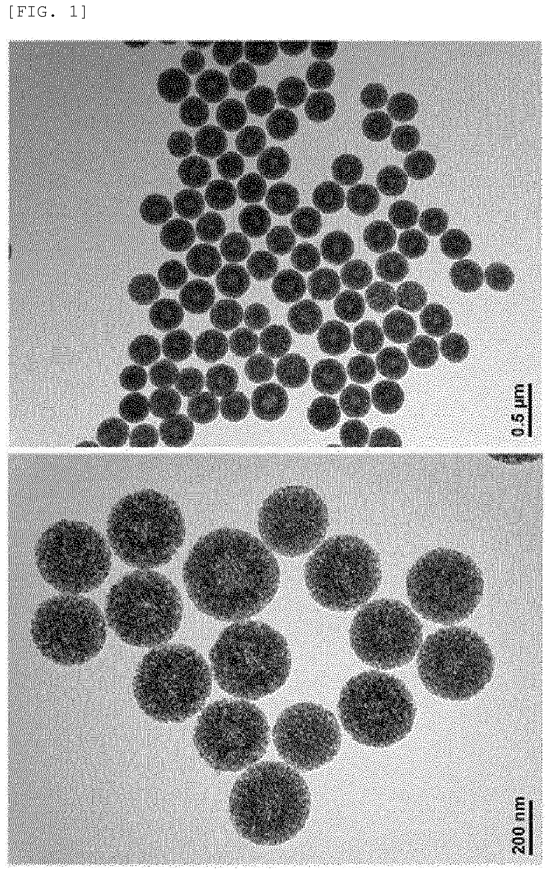

Small pore particles and porous silica particles prepared in Experimental Examples 1-1-(1) to (3) were observed under a microscope to determine whether the small pore particles were uniformly formed, or the pores were sufficiently expanded to uniformly form the porous silica particles (FIGS. 1 to 4).

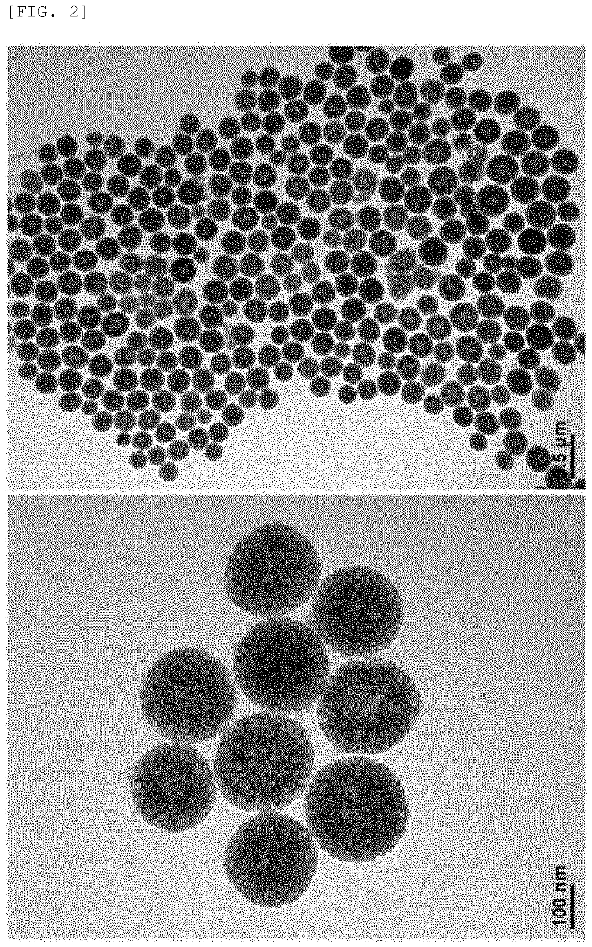

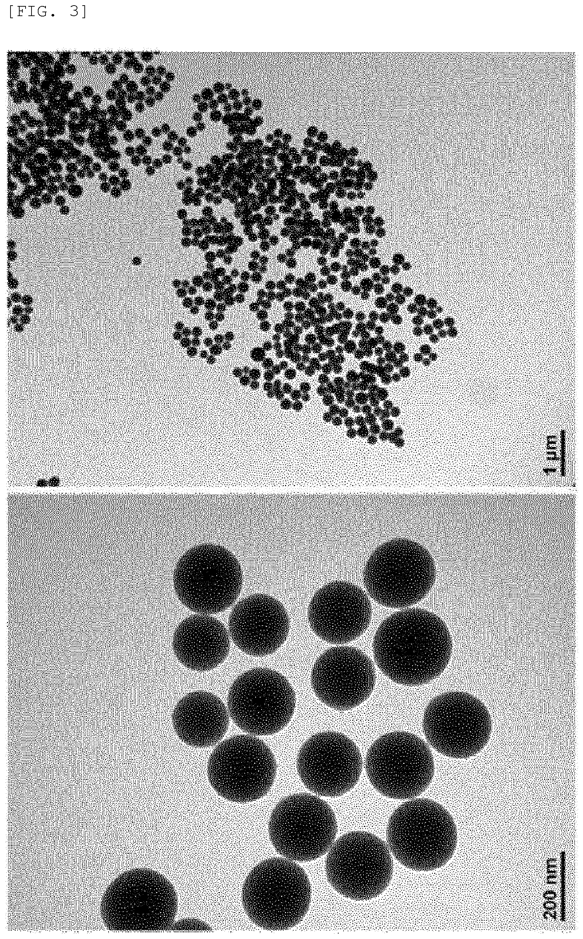

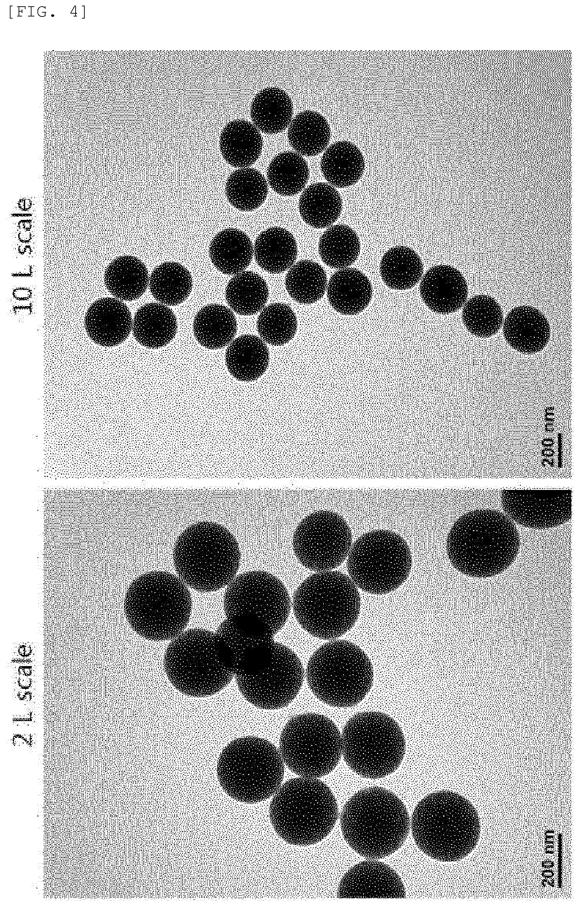

FIG. 1 is photographs of the porous silica particles in Example 1-1-(1), and FIG. 2 is photographs of the porous silica particles in Example 1-1-(2), and from these drawings, it can be seen that spherical porous silica particles having sufficiently expanded pores were formed evenly. FIG. 3 is photographs of the small pore particles in Example 1-1-(1), and FIG. 4 is comparative photographs of the small pore particles in Examples 1-1-(1) and 1-1-(3), and from these drawings, it can be seen that spherical small pore particles were formed evenly.

**4. Calculation of pore diameter and BET surface area**

Surface areas of the small pore particles in Example 1-1-(1) and the porous silica particles of Examples 1-1-(1), (7), (8), (10) and (11) were calculated. The surface areas were calculated by Brunauer-Emmett-Teller (BET) method, and the pore size distributions were calculated by Barrett-Joyner-Halenda (BJH) method.

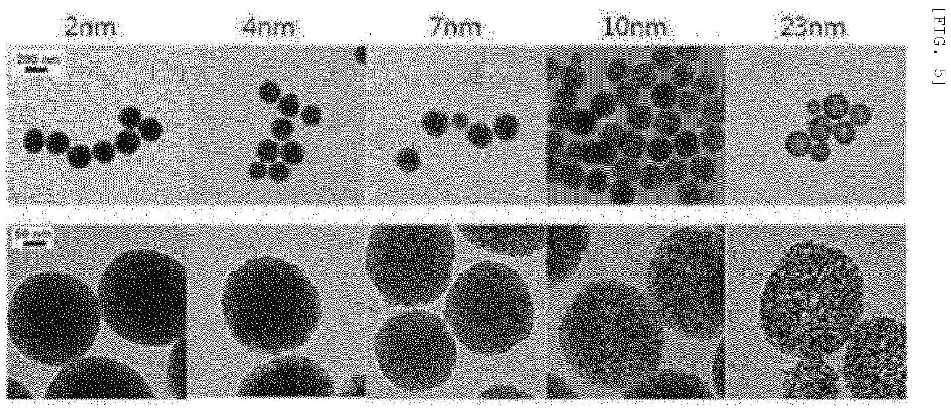

Micrographs of the particles are shown in FIG. 5, and the calculation results are shown in Table 1 below.

[TABLE 1]

| Particle classification | Pore diameter (nm) | BET surface area ($m^2/g$) |
|---|---|---|
| Small pore particles of 1-1-(1) | 2.1 | 1,337 |
| 1-1-(7) | 4.3 | 630 |
| 1-1-(8) | 6.9 | 521 |
| 1-1- (1) | 10.4 | 486 |
| 1-1-(10) | 23 | 395 |
| 1-1-(11) | 12.3 | 379 |

**5. Identification of biodegradability**

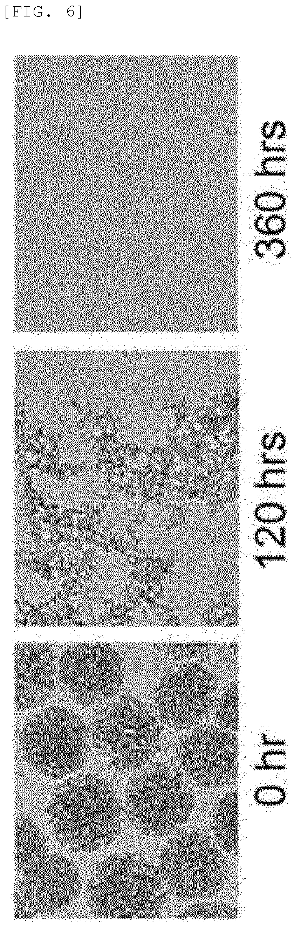

In order to identify biodegradability of the porous silica particles in Example 1-1-(1), biodegradability at 37 °C in SBF (pH 7.4) was observed under a microscope at 0 hours, 120 hours and 360 hours, and results thereof are shown in FIG. 6. Referring to FIG. 6, it can be seen that the porous silica particles are biodegraded and almost degraded after 360 hours.

**6. Measurement of absorbance ratio**

Absorbance ratio over time was measured according to Equation 1 below.

[Equation 1]

$$A_t/A_0$$

(wherein $A_0$ is absorbance of the porous silica particles measured by putting 5 mL of suspension containing 1 mg/ml of the porous silica particles into a cylindrical permeable membrane having pores with a pore diameter of 50 kDa,

15 mL of the same solvent as the suspension comes into contact with an outside of the permeable membrane, and the inside/outside of the permeable membrane are horizontally stirred at 60 rpm and 37 °C, and

$A_t$ indicates absorbance of the porous silica particles measured after lapse of "t" hours since $A_o$ was measured).

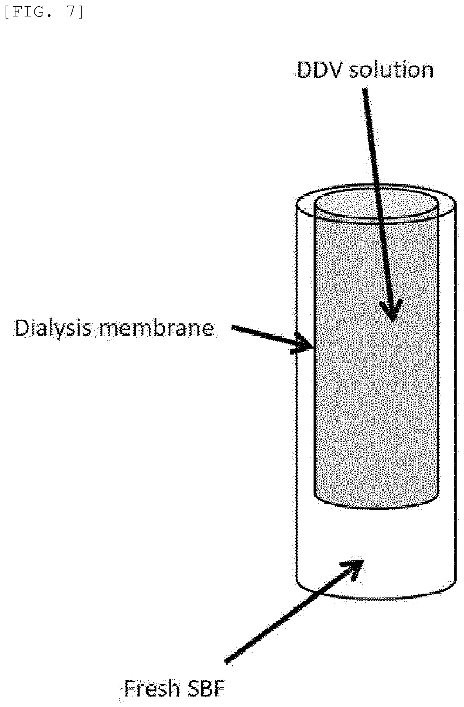

Specifically, 5 mg of porous silica particle powder was dissolved in 5 mL of SBF (pH 7.4). Thereafter, 5 mL of porous silica particle solution was placed in a permeable membrane having pores with a pore diameter of 50 kDa shown in FIG. 7. 15 mL of SBF was added to an outer membrane, and the SBF on the outer membrane was replaced every 12 hours. Degradation of the porous silica particles was performed at 37 °C under horizontal stirring at 60 rpm.

Then, the absorbance was measured by UV-vis spectroscopy and analyzed at $\lambda$ = 640 nm.

**(1) Measurement of absorbance ratio**

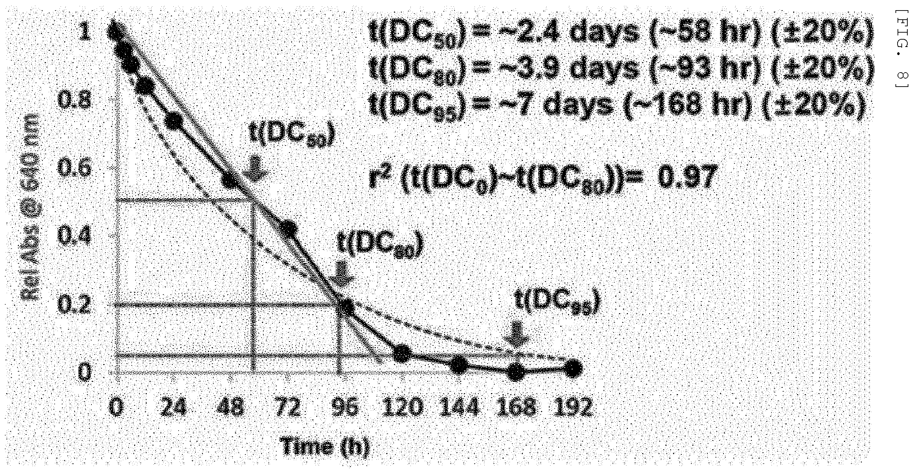

Absorbance ratio of the porous silica particles in Example 1-1-(1) was measured according to the above method, and results thereof are shown in FIG. 8.

Referring to FIG. 8, it can be seen that t, at which the absorbance ratio becomes 1/2, is about 58 hours to demonstrate very slow degradation.

**(2) Particle size**

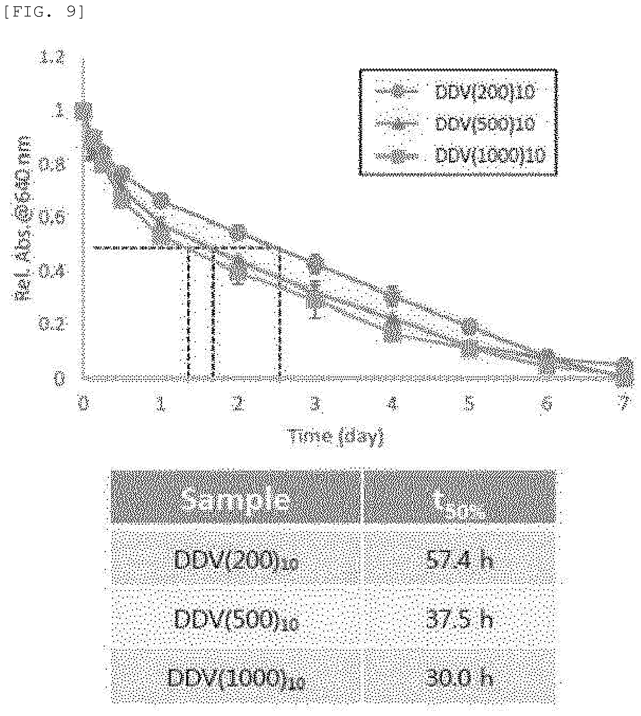

Absorbances of the porous silica particles in Examples 1-1-(1), (5) and (6) were measured according to Equation 1 above, and results thereof are shown in FIG. 9 (SBF used as the suspension and the solvent).

Referring to FIG. 9, it can be seen that t is decreased as the particle size is increased.

**(3) Average pore diameter**

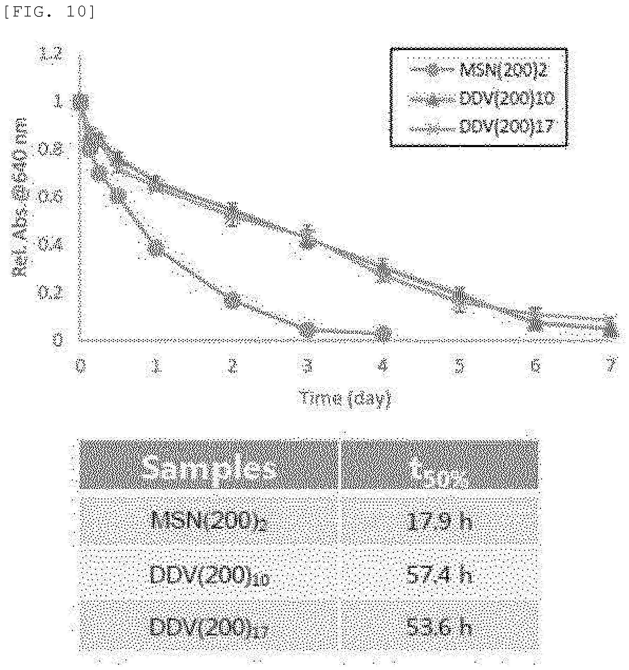

Absorbances of the porous silica particles in Examples 1-1-(1) and (9) and the microporous silica particles in Example 1-1-(1) as a control were measured according to Equation 1 above, and results thereof are shown in FIG. 10 (SBF used as the suspension and the solvent).

Referring to FIG 10, it can be seen that the porous silica particles of the inventive example have a significantly larger t than the control.

**(4) pH**

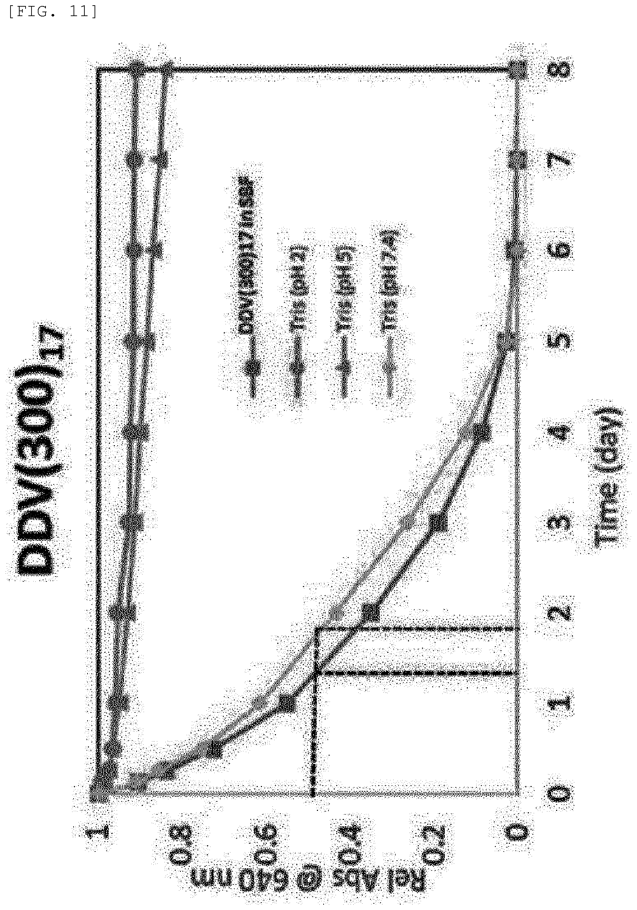

Absorbance of the porous silica particles in Example 1-1-(4) for each pH was measured. The absorbance was measured in SBF and in Tris at pH 2, 5, and 7.4, and results thereof are shown in FIG. 11.

Referring to FIG 11, it could be seen that, although there is a difference in t in relation to pH, t at which all absorbance ratio becomes 1/2 was 20 or more.

**(5) Charging**

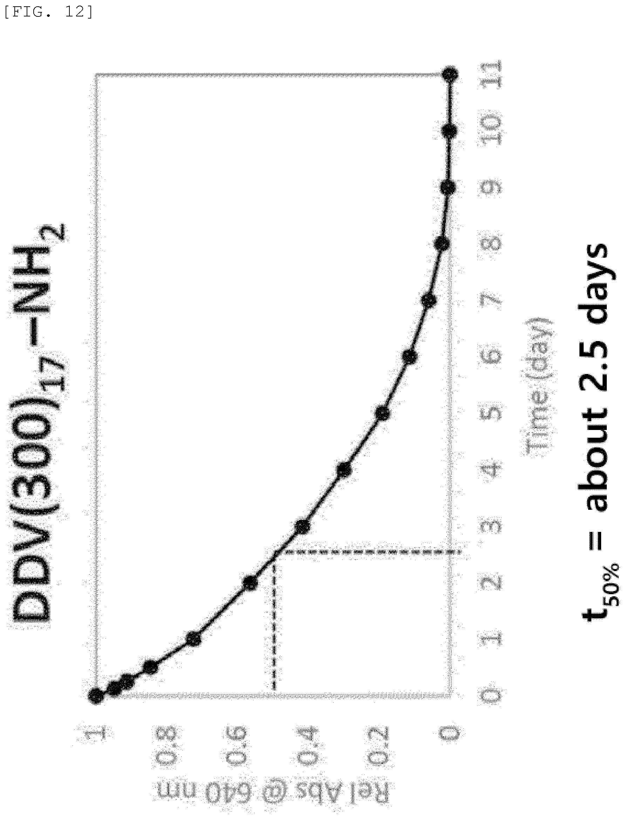

Absorbance of the porous silica particles in Example 1-2-(1) was measured, and results thereof are shown in FIG. 12 (Tris (pH 7.4) used as the suspension and the solvent).

Referring to FIG 12, it could be seen that t at which the absorbance ratio of the positively charged particles becomes 1/2 was 20 or more.

**Example 2. Selection of mRNA nucleotide sequence and carrying on carrier**

**1. Selection of mRNA nucleotide sequence**

Six sequences, Seq 1 to Seq 6 in Table 2 below, were selected while changing the nucleotide sequence encoding the spike protein of the coronavirus and the sequence of the nucleic acid linking thereto. Table 2 shows the mRNA configuration including the viral receptor and the sequence linked thereto. The atgttcgtgttcctggtgctgctgctgcctctggtgtccagccagtgtgtg nucleotide sequence of SEQ ID NO: 6 was used for the signal peptide (SP), the nucleotide sequence of SEQ ID NO: 2, which is the RBD sequence of COVID-19, was used for the corresponding RBD, and the nucleotide sequence of SEQ ID NO: 12 was used for the whole spike protein.

[TABLE 2]

| Product | Configuration | NO. |
|---|---|---|
| Seq. 1 | *SP-*RBD-tgatga | SEQ ID NO: 7 |
| Seq. 2 | SP-RBD-ggctacatccctgaagcgcctagagacggccagtgctacgtgagatgcgacggcgaatgggtgctgctgtccactttcctgtgatga | SEQ ID NO: 8 |
| Seq. 3 | SP-RBD-ggctctggttctggctctggctacatccctgaagcgcctagagacggccagtgctacgtgagatgcgacggcgaatgggtgctgctgtccactttcctgtgatga | SEQ ID NO: 9 |
| Seq. 4 | SP-RBD-gaagccgccgccaaggaagccgccgccaaggaagccgccgccaaggaagccgccgccaaggaagccgccgccaagggctacatccctgaagcgcctagagacggccagtgctacgtgagatgcgacggcgaatgggtgctgctgtccactttcctgtgatga | SEQ ID NO: 10 |

(continued)

| Product | Configuration | NO. |
|---|---|---|
| Seq. 5 | SP-RBD-tccggcggcggctacatccctgaagcgcctagagacggccagtgctacgtgagatgcgacggcgaatgggtgctgctgtccactttcctgtgatga | SEQ ID NO: 11 |
| Seq. 6 | Whole spike protein | SEQ ID NO: 12 |
| *SP: Signal peptide, *RBD: Receptor-binding domain | | |

**2. Preparation of mRNA-DegradaBALL**

**(1) mRNA-DegradaBALL with a weight ratio of 1:10**

DegradaBALL, in which the porous silica particles of Example 1-1-(11) above were subjected to surface modification as in Example 1-2-(1) above, was used as a carrier.

After mixing 150 μg of the carrier powder with 15 μg of each mRNA sequence of Seq. 1 to Seq. 6 of Example 2-1 above, the mixture was dissolved in 50 μL of PBS and gently shaken to prepare mRNA-DegradaBALL.

**(2) mRNA-DegradaBALL with a weight ratio of 1:8**

mRNA-DegradaBALL with a weight ratio of mRNA: DegradaBALL of 1:8 was prepared by adopting the same manner as in Example 2-2-(1) except that the dose of DegradaBALL was changed to 120 μg.

**3. Determination of weight ratio of mRNA-DegradaBALL**

The mRNA-DegradaBALLs prepared in Examples 2-2-(1) and (2) above were stored at room temperature for 30 minutes, and then centrifuged at 13,000 rpm for 5 minutes.

The supernatant was obtained from the centrifuged solution, and the absorbance was measured at λ = 260 nm.

The amount of mRNA carried on the DegradaBALL was calculated based on an amount of mRNA remaining in the supernatant.

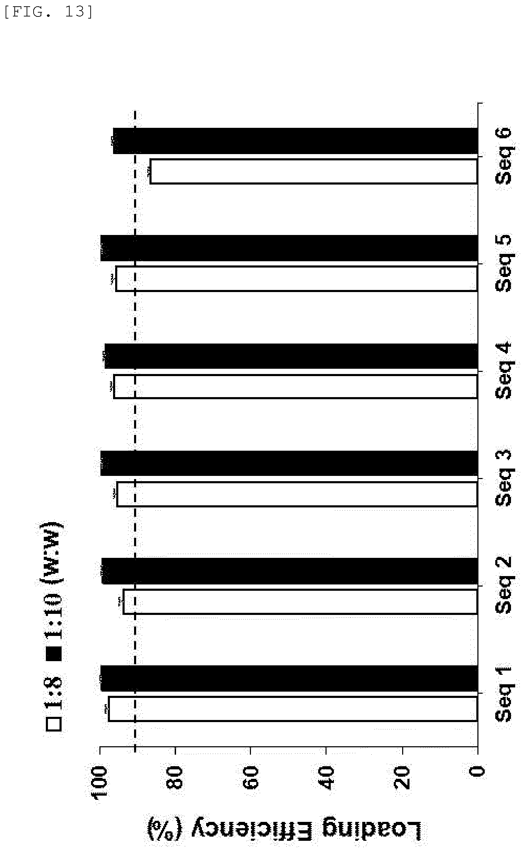

As a result, it was confirmed that 80% or more of mRNA was carried on the DegradaBALL, regardless of the weight of DegradaBALL powder mixed with all of Seq. 1 to Seq. 6. However, when mixed with 150 μg of DegradaBALL, it was confirmed that 90% or more of mRNA was carried on the DegradaBALL to exhibit higher loading efficiency, and thereby setting it as optimal conditions for carrying the mRNA (FIG. 13).

**4. Stability test of DegradaBALL**

The mRNA-DegradaBALL of Example 2-2-(1) above was put into 2% or 4% sucrose-PBS at a temperature condition of - 20 °C or -70 °C, respectively, then stored for 7 days.

Changes in mRNA loading amount were confirmed by measuring absorbance at λ=260 nm before and after storage, respectively.

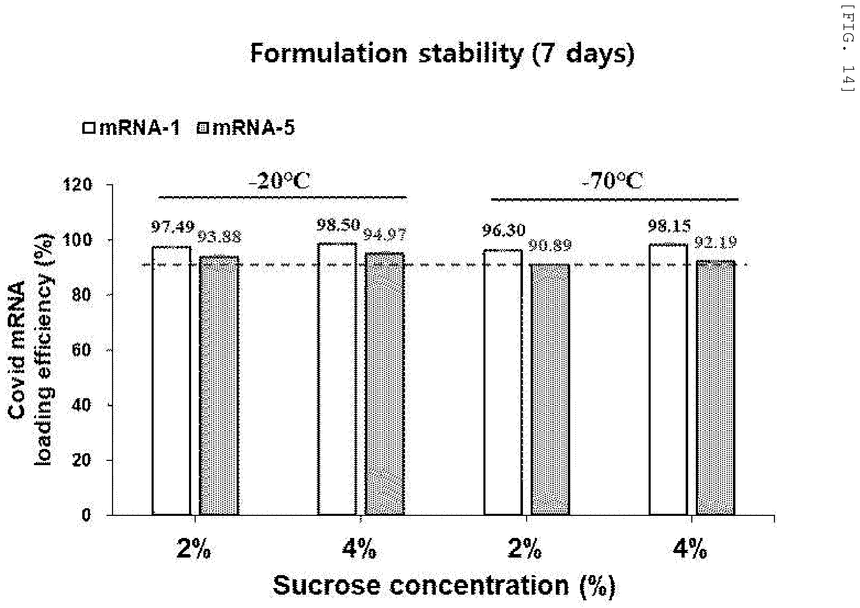

As a result, it was confirmed that 90% or more of the carried mRNA was maintained regardless of sucrose concentration, storage temperature, or mRNA sequence, thereby confirming that DegradaBALL might stably carry mRNA (FIG. 14).

**5. Translation efficiency according to mRNA nucleotide sequence**

**(1) in *vitro***

All cells were cultured in a $CO_2$ incubator (SANYO Electric, Osaka, Japan) at 37 °C under 5% $CO_2$ condition. A549 cells were cultured in F-12K medium containing 10 % FBS (v/v) and 10 unit/mL of penicillin/streptomycin. CT26 cells were cultured in RPMI-1640 medium containing 10 % FBS (v/v) and 10 unit/mL of penicillin/streptomycin.

$5 \times 10^4$ of the A549 cells were dispensed in a 24-well plate, and mRNA-DegradaBALL in which 1 μg of each mRNA of Example 2-1 above and 10 μg of DegradaBALL obtained by surface-modifying the porous silica particles of Example 1-1-(11) above as in Example 1-2(1) above were mixed; or, Seq. 1 not carried on the carrier was injected into each cell, and transfection was performed in serum-free F-12K medium for 6 hours. Then, the medium was replaced with a growth medium and further cultured for 18 or 32 hours.

After culturing, the cells were separated and centrifuged at 2,500 rpm for 10 minutes, the supernatant was taken, and the translation efficiency of RBD was analyzed through Elisa (SARS-CoV-2 RBD ELISA kit, ab289833).

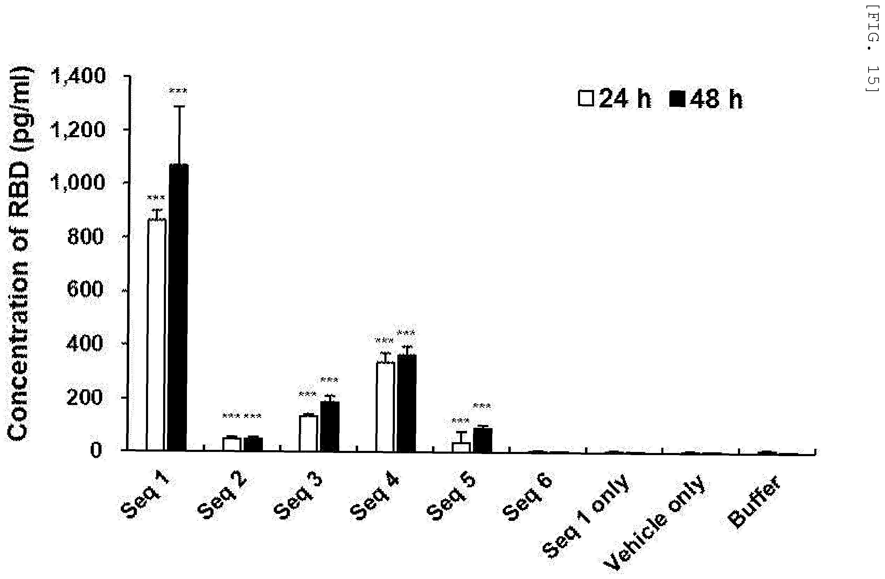

As a result, it was confirmed that RBD was translated significantly in all of Seq. 1 to Seq. 5, and among them, it was verified that Seq. 1 translated the RBD to the highest level (FIG. 15).

**(2) in *vivo***

All animal experiments below proceeded according to the Institutional Animal Care and Use Committee (IACUC) of Seoul National University. Balb/C female mice were purchased from ORIENT BIO (Seongnam, Korea). During an experimental period, the animals were kept under environmental conditions of a temperature of 19 to 25 °C, a humidity of 40 to 70% and a cycle of light-12 hours and dark-12 hours.

The mRNA-DegradaBALL of Example 2-5-(1) above was dissolved in 100 μL of sterilized PBS and delivered to Balb/C mice by submuscular injection, respectively. After inducing a primary immune response for 2 weeks, serum was collected, and the concentration of IgG specifically bound to SARS-CoV-2 S1-RBD was measured by ELISA. The measurement by ELISA was performed using a method known to those skilled in the art (Abcam, ab275300).

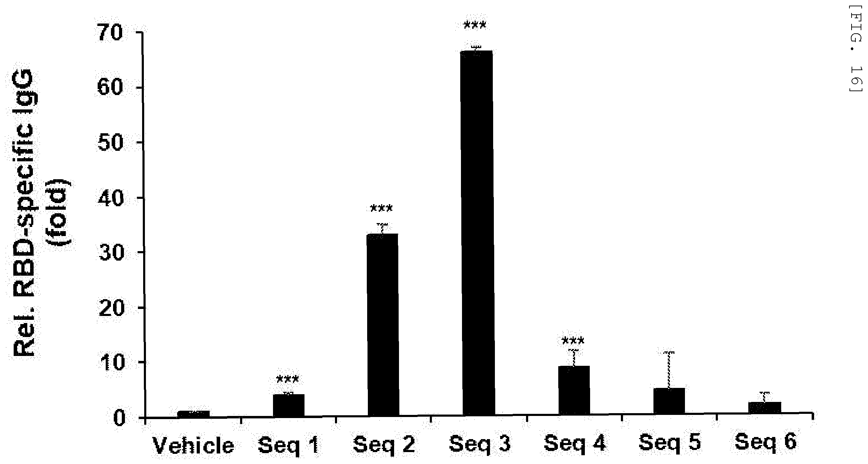

As a result, it was confirmed that the most SARS-CoV-2 S1-RBD-specific IgG was detected in mice injected with Seq. 3 mRNA carried on DegradaBALL (FIG. 16). Based on the above results, Seq. 3 was selected as an mRNA sequence exhibiting high vaccine efficiency at the actual *in vivo* level.

**Example 3. Drug dose range and efficacy test**

**1. Drug dose range and administration cycle**

0 μg (lane 1), 7.5 μg (lane 2), 15 μg (lane 3), and 45 μg (lane 4) of Seq. 3 were carried on 0 μg (lane 1), 75 μg (lane 2), 150 μg (lane 3), and 450 μg (lane 4) of DegradaBALL obtained by surface-modifying the porous silica particles of Example 1-1-(11) above as in Example 1-2(1) above, respectively, and dissolved in 100 μL of phosphate buffered saline (PBS), and then injected submuscularly to Balb/C mice at 0 week, and 2nd week.

One week after the final injection, serum was collected from the mice, and the concentration of IgG specifically bound to SARS-CoV-2 S1-RBD was measured by ELISA (Abcam, ab275300).

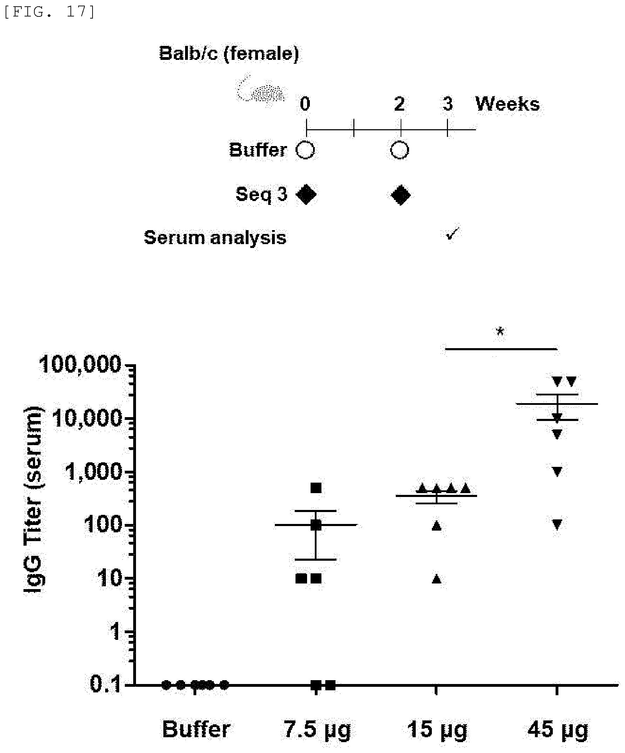

As a result, in all experimental groups administered with Seq. 3, it was observed that IgG binding to RBD was increased in proportion to the administered dose (FIG. 17).

Based on the results in which IgG production was also significantly increased in the group treated with a high dose of 45 μg compared to the groups treated with other doses, the administration dose and cycle were determined that 45 μg of Seq. 3 would be administered in a 2-week interval.

**2. Confirmation of concentration of binding antibody**

45 μg of Seq. 3 was carried on the DegradaBALL of Example 3-1 above, and dissolved in 100 μL of phosphate buffered saline (PBS), and then injected submuscularly to Balb/C mice at 0 week, and 2nd week.

Serum was collected at the point of 2, 3, and 5 weeks after the initial injection, and the concentration of IgG specifically bound to SARS-CoV-2 S1-RBD was measured by ELISA (Covid-19 S1 RBD protein Human IgG Elisa Kit, RayBiotech, IEQ-CoVSN-IgG-1; Goat Anti-Mouse IgG Fc(biotin), Abcam).

**3. Confirmation of concentration of neutralizing antibody**

To confirm the neutralizing ability of the antibody, a surrogate virus neutralization test (sVNT) was performed with the serum collected in Example 3-2.

Specifically, sVNT was analyzed by ELISA method using the SARS-CoV-2 Spike RBD-ACE2 Blocking Antibody Detection ELISA Kit (cell signaling technology) product.

As a result, SARS-COV-2 S1-RBD-specific IgG was detected even in the group (lane 2) after lapse of 2 weeks from the initial injection, but exhibited a neutralizing activity of 16 to 42% (average 30.56%).

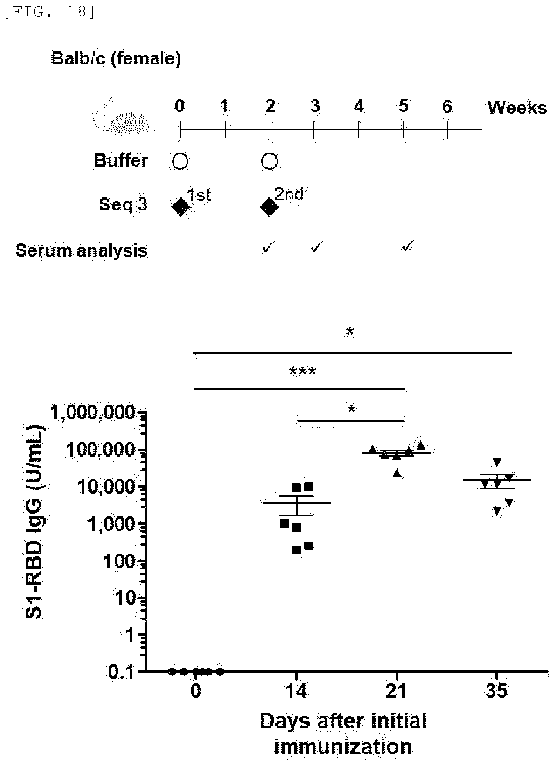

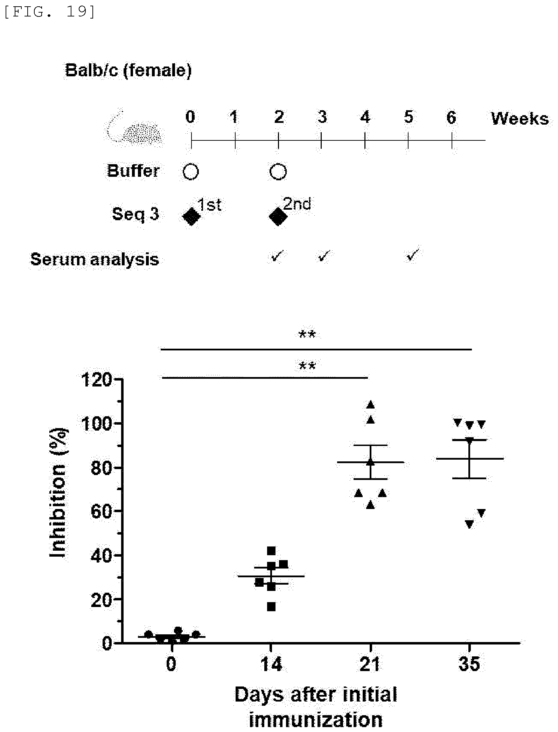

On the other hand, in the remaining experimental groups after lapse of 3 and 5 weeks, significantly higher levels of IgG specifically bound to SARS-COV-2 SL-RBD were detected than the 2-week lapsed group, and in the sVNT experiment, the 3-week lapsed experimental group and the 5-week lapsed experimental group exhibited significant difference in the neutralizing ability of about 63 to 108% (average 82.31%), and neutralizing ability of about 53 to 100% (average 83.89%), respectively (FIGS. 18 and 19).

**4. Confirmation of expression duration and expression location**

In order to confirm the expression location and expression duration of mRNA when mRNA-DegradaBALL was delivered to mice, the DegradaBALL of Example 3-1 carrying 10 μg of Fluc-encoding reporter mRNA was dissolved in 100 μL of PBS, and delivered to Balb/C mice by submuscular or intratumoral injection (IT).

For the analysis of intratumoral injection, tumor-bearing mice (Balb/C) were prepared by subcutaneous injection of CT26 cells ($1 \times 10^6$) in 100 μL sterile 1 × PBS solution containing Matrigel. When the tumor volume reached about 200 mm3, DegradaBALL carrying the reporter mRNA or DegradaBALL not carrying the mRNA was injected into the tumor location of the mice, respectively.

Thereafter, 100 μL of Luciferin solution (0.3 mg/mL in DPBS) was administered intraperitoneally to the mice over time (6 hours, 1 day, 2 days, 4 days and 7 days), and after 1 minute of administration, the location and intensity of luminescence were analyzed through an *in vivo* optical imaging system (IVIS). On the 7th day after injection, tumor, lung, heart, kidney, liver, and thymus were collected and the level of luminescence was determined.

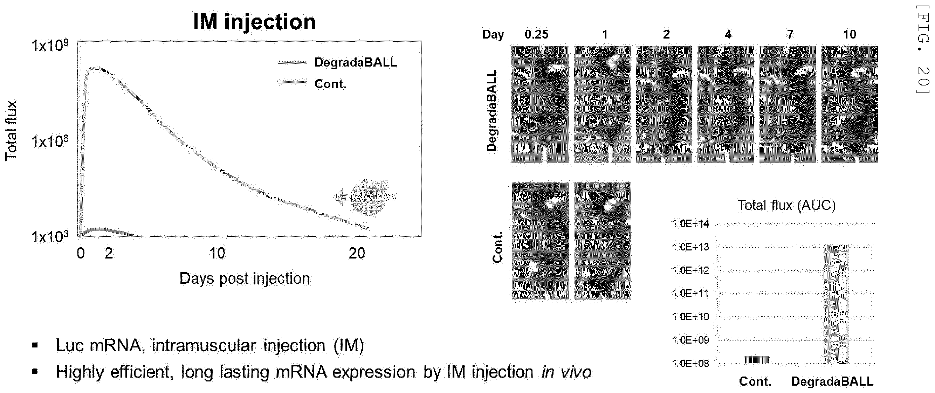

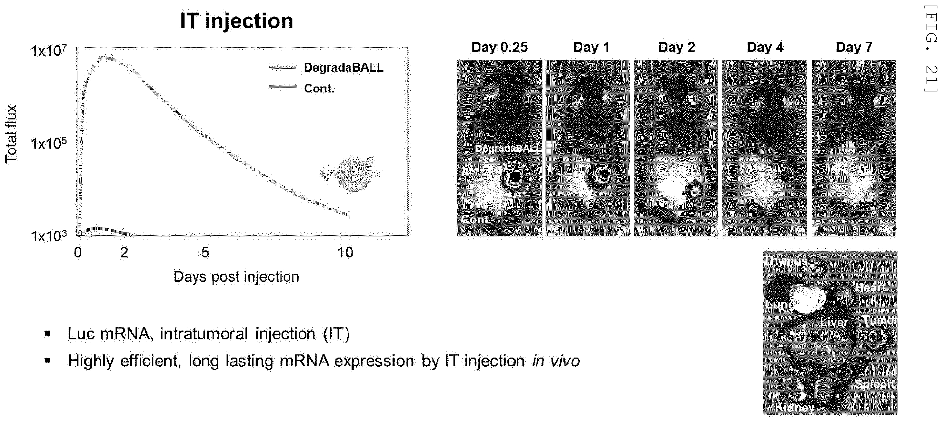

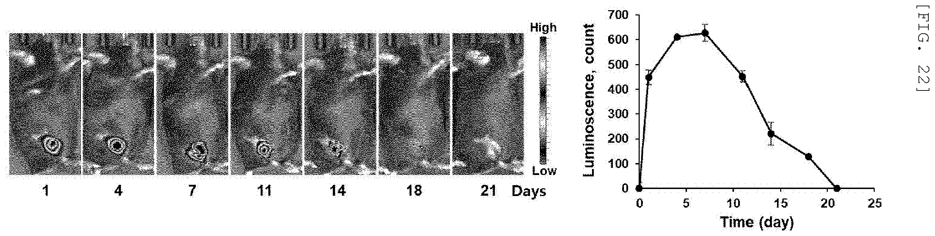

As a result, it was confirmed that, when injected submuscularly, fluorescence was significantly emitted near the injection site even 10 days after injection, and no signal was observed in other tissues other than the injection site (FIGS. 20 and 22). Likewise, it was also confirmed that luciferase was still expressed even after about 7 days even when injected intratumorally, and any signal was not observed in organs other than the tumor tissue (FIG. 21). Through the corresponding experimental data, it was confirmed that the carried mRNA was efficiently accumulated at the desired delivery site by the DegradaBALL, and did not move to other organs other than the injection site, as well as continuous expression might be induced by releasing the carried mRNA over a long period of time.

5. Comparison of efficiency between mRNA-LNP and DegradaBALL-mRNA

An experiment was performed using the same method as the experimental method for confirming whether to produce binding antibody/neutralizing antibody in Example 3-2 and Example 3-3, and expression duration and expression location in Example 3-4, except that the mRNA was carried on LNP (invivofectamine 3.0 Reagent, Thermo Fischer) rather than DegradaBALL, and the *in vivo* expression duration, expression site after submuscular injection, production level of the binding antibody, and production level of the neutralizing antibody were compared to the case of using mRNA-DegradaBALL in Example 2-2.

[TABLE 3]

| Division | mRNA-LNP | mRNA-DegradaBALL |
|---|---|---|
| Expression duration *in vivo* | About 1 day | About 21 days |
| Expression site after IM administration | Liver (90%), injection site (10%) | Injection site (90%), secondary lymphoid organ (10%) |
| Whether to produce binding antibody | +++++ | +++++ |
| Whether to produce neutralizing antibody | ++ | ++++ |

As shown in Table 3 below, it was confirmed that, when delivered *in vivo* in the form of mRNA-LNP, the expression duration is relatively short, about 1 day, compared to the case of carrying on the DegradaBALL of the present invention, and a ratio of delivery to other tissues was high, for example, about 90% was expressed in the liver other than the injection site, etc., as well as the amount of neutralizing antibodies produced, which prevent the viral antigen from binding to the target receptor, was low.

**6. Stability test of DegradaBALL *in vivo***

The mRNA-DegradaBALL of Example 2-2 above was stored at 4 °C for 2 weeks and then injected submuscularly to the mice by the same method as Example 3-4 above, and whether or not fluorescence is emitted and location thereof was determined after 6 and 48 hours.

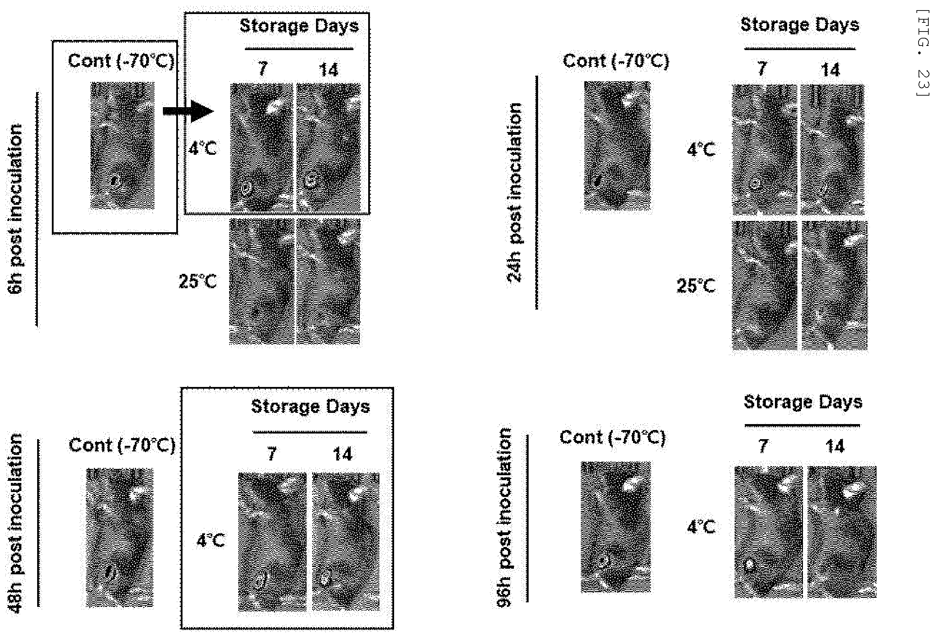

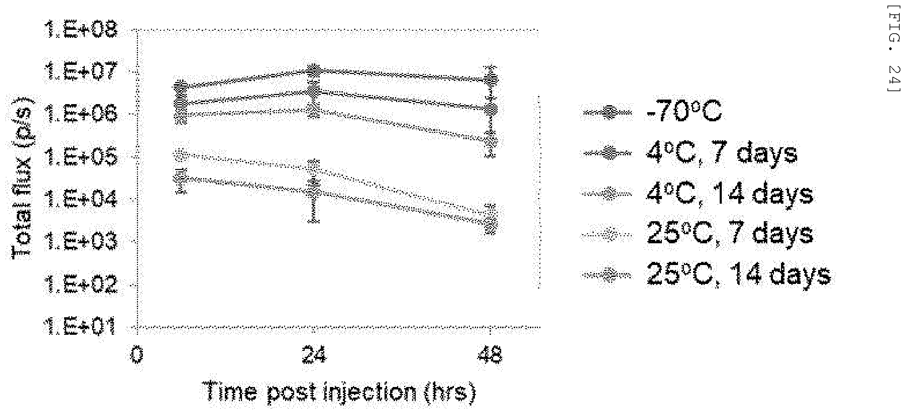

As a result, it was confirmed that even when stored at 4°C for 2 weeks, there was no difference in the expression level based on 6 hours after injection compared to DegradaBALL stored at -70°C, and a very high level of expression was observed even after lapse of 48 hours from injection (FIGS. 23 and 24).

## Claims

1. A nucleic acid molecule of the following sequence:

RBD-(L)n-X

(wherein, RBD is a sequence of at least a partial region including a receptor-binding domain in a spike protein, L is a linker sequence, n is 0 or 1, and X is the nucleotide sequence of SEQ ID NO: 1).

2. The nucleic acid molecule according to claim 1, wherein the RBD is a virus-derived sequence forms a triplet by the receptor-binding domain thereof.

3. The nucleic acid molecule according to claim 1, wherein the RBD has a length of 100 nt to 5,000 nt.

4. The nucleic acid molecule according to claim 1, wherein the RBD is the nucleotide sequence of SEQ ID NO: 2.

5. The nucleic acid molecule according to claim 1, wherein L is the nucleotide sequence of SEQ ID NO: 3 or SEQ ID NO: 4.

6. The nucleic acid molecule according to claim 1, wherein the RBD is a virus-derived sequence selected from the group consisting of *herpesviridae, orthomyxoviridae, rhabdoviridae, paramyxoviri dae, papilomaviridae, adenoviridae, parvoviridae, astrovirid ae, reoviridae, bunyaviridae, arteriviridae, caliciviridae, hepeviridae, bornaviridae, arenaviridae, togaviridae, filoviridae, retroviridae, flaviv iridae* and *coronaviridae.*

7. The nucleic acid molecule according to claim 1, wherein the RBD is a virus-derived sequence selected from the group consisting of *Colacovirus, Decacovirus, Duvinacovirus, Luchavirus, Minacovirus, Minunacovirus, Myota covirus, Nyctacovirus, Pedacovirus, Rhinacovirus, Setracovir us, Soracovirus, Tegacovirus, Embecovirus, Hibecovirus, Merb ecovirus, Nobecovirus, Sarbecovirus, Brangacovirus, Cegacovi rus, Igacovirus, Andecovirus, Buldecovirus* and *Herdecovirus.*

8. A virus vaccine composition comprising the nucleic acid molecule according to any one of claims 1 to 7.

9. The virus vaccine composition according to claim 8, wherein the nucleic acid molecule is carried on a carrier selected from the group consisting of a viral carrier, virus-like particles (VLPs), positively charged polymer, liposome, lipid nanoparticles, gold, semiconductor quantum dots, carbon nanotube and porous silica particles.

10. The virus vaccine composition according to claim 8, wherein the nucleic acid molecule is carried on porous silica particles.

11. The virus vaccine composition according to claim 10, wherein the porous silica particles have an average pore diameter of 5 nm to 100 nm.

12. The virus vaccine composition according to claim 10, wherein the porous silica particles are positively charged inside the pores.

13. The virus vaccine composition according to claim 10, wherein the porous silica particles are biodegradable particles.

14. The virus vaccine composition according to claim 10, wherein a weight ratio of the nucleic acid molecule to the porous silica particles is 1:1 to 30.

[FIG. 1]

0.5 μm
200 nm

[FIG. 2]

5 μm
100 nm

[FIG. 3]

1 μm
200 nm

[FIG. 4]

10 L scale
200 nm
2 L scale
200 nm

[FIG. 5]

23nm
10nm
7nm
4nm
2nm

[FIG. 6]

360 hrs
120 hrs
0 hr

[FIG. 7]

DDV solution
Dialysis membrane
Fresh SBF

[FIG. 8]

$t(DC_{50})$ = ~2.4 days (~58 hr) (±20%)
$t(DC_{80})$ = ~3.9 days (~93 hr) (±20%)
$t(DC_{95})$ = ~7 days (~168 hr) (±20%)
$r^2$ ($t(DC_0)$~$t(DC_{80})$)= 0.97
$t(DC_{50})$
$t(DC_{80})$
$t(DC_{95})$
Rel Abs @ 640 nm
1
0.8
0.6
0.4
0.2
0
0 24 48 72 96 120 144 168 192
Time (h)

[FIG. 9]

1.2
1
0.8
0.6
0.4
0.2
0
Rel. Abs. @650 nm
0
1
2
3
4
5
6
7
Time (day)
DDV(200)10
DDV(500)10
DDV(1000)10


| Sample | $t_{50\%}$ |
|---|---|
| $DDV(200)_{10}$ | 57.4 h |
| $DDV(500)_{10}$ | 37.5 h |
| $DDV(1000)_{10}$ | 30.0 h |

[FIG. 10]

MSN(200)2
DDV(200)10
DDV(200)17
Rel. Abs. @640 nm
Time (day)
1.2
1
0.8
0.6
0.4
0.2
0
0
1
2
3
4
5
6
7

| Samples | $t_{50\%}$ |
|---|---|
| $MSN(200)_2$ | 17.9 h |
| $DDV(200)_{10}$ | 57.4 h |
| $DDV(200)_{17}$ | 53.6 h |

[FIG. 11]

DDV(300)17
Rel Abs @ 640 nm
1
0.8
0.6
0.4
0.2
0
0
1
2
3
4
5
6
7
8
Time (day)
DDV(300)17 in SBF
Tris (pH 2)
Tris (pH 5)
Tris (pH 7.4)

[FIG. 12]

DDV(300)$_{17}$-NH$_2$
Rel Abs @ 640 nm
1
0.8
0.6
0.4
0.2
0
0 1 2 3 4 5 6 7 8 9 10 11
Time (day)
$t_{50\%}$ = about 2.5 days

[FIG. 13]

□ 1:8 ■ 1:10 (W:W)
Loading Efficiency (%)
100
80
60
40
20
0
Seq 1
Seq 2
Seq 3
Seq 4
Seq 5
Seq 6

[FIG. 14]

Formulation stability (7 days)
mRNA-1
mRNA-5
-20°C
-70°C
97.49
93.88
98.50
94.97
96.30
90.89
98.15
92.19
2%
4%
2%
4%
Sucrose concentration (%)
Covid mRNA loading efficiency (%)
0
20
40
60
80
100
120

[FIG. 15]

□24 h ■48 h
Concentration of RBD (pg/ml)
0
200
400
600
800
1,000
1,200
1,400
Seq 1
Seq 2
Seq 3
Seq 4
Seq 5
Seq 6
Seq 1 only
Vehicle only
Buffer

[FIG. 16]

Rel. RBD-specific IgG
(fold)
70
60
50
40
30
20
10
0
Vehicle
Seq 1
Seq 2
Seq 3
Seq 4
Seq 5
Seq 6
***
***
***
***

[FIG. 17]

Balb/c (female)
0
2
3
Weeks
Buffer
Seq 3
Serum analysis

*
100,000
10,000
1,000
100
10
1
0.1
IgG Titer (serum)
Buffer
7.5 µg
15 µg
45 µg

[FIG. 18]

Balb/c (female)
0 1 2 3 4 5 6 Weeks
Buffer
Seq 3
1st
2nd
Serum analysis

*
***
*
S1-RBD IgG (U/mL)
1,000,000
100,000
10,000
1,000
100
10
1
0.1
0
14
21
35
Days after initial immunization

[FIG. 19]

Balb/c (female)
0
1
2
3
4
5
6
Weeks
Buffer
Seq 3
1st
2nd
Serum analysis

**
**
Inhibition (%)
120
100
80
60
40
20
0
0
14
21
35
Days after initial immunization

[FIG. 20]

IM injection
DegradaBALL
Cont.
Total flux
1x10^9
1x10^6
1x10^3
0
2
10
20
Days post injection
Day
0.25
1
2
4
7
10
DegradaBALL
Cont.
Total flux (AUC)
1.0E+14
1.0E+13
1.0E+12
1.0E+11
1.0E+10
1.0E+09
1.0E+08
Cont.
DegradaBALL
Luc mRNA, intramuscular injection (IM)
Highly efficient, long lasting mRNA expression by IM injection in vivo

[FIG. 21]

IT injection
Total flux
1x10^7
1x10^5
1x10^3
0
2
5
10
Days post injection
DegradaBALL
Cont.
Day 0.25
Day 1
Day 2
Day 4
Day 7
DegradaBALL
Cont.
Thymus
Heart
Tumor
Lung
Liver
Spleen
Kidney
▪ Luc mRNA, intratumoral injection (IT)
▪ Highly efficient, long lasting mRNA expression by IT injection in vivo

[FIG. 22]

High
Low
1
4
7
11
14
18
21
Days
Luminoscence, count
Time (day)

[FIG. 23]

6h post inoculation
Cont (-70°C)
Storage Days
7
14
4°C
25°C
24h post inoculation
Cont (-70°C)
Storage Days
7
14
4°C
25°C
48h post inoculation
Cont (-70°C)
Storage Days
7
14
4°C
96h post inoculation
Cont (-70°C)
Storage Days
7
14
4°C

[FIG. 24]

-70°C
4°C, 7 days
4°C, 14 days
25°C, 7 days
25°C, 14 days
Total flux (p/s)
1.E+08
1.E+07
1.E+06
1.E+05
1.E+04
1.E+03
1.E+02
1.E+01
0
24
48
Time post injection (hrs)

**INTERNATIONAL SEARCH REPORT**

International application No. **PCT/KR2022/007697**

**A. CLASSIFICATION OF SUBJECT MATTER**

**C07K 19/00**(2006.01)i; **C07K 14/005**(2006.01)i; **A61K 39/12**(2006.01)i; **A61K 9/51**(2006.01)i; **A61P 31/14**(2006.01)i

According to International Patent Classification (IPC) or to both national classification and IPC

**B. FIELDS SEARCHED**

Minimum documentation searched (classification system followed by classification symbols)

C07K 19/00(2006.01); A61K 9/00(2006.01); A61K 9/14(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 수용체 결합 도메인(receptor binding domain), 스파이크(spike), 바이러스(virus), 백신(vaccine), 다공성 실리카(porous silica), 전달(delivery)

**C. DOCUMENTS CONSIDERED TO BE RELEVANT**

| Category* | Citation of document, with indication, where appropriate, of the relevant passages | Relevant to claim No. |
|---|---|---|
| A | JEONG, Hyein et al. A novel DNA vaccine against SARS-CoV-2 encoding a chimeric protein of its receptor-binding domain (RBD) fused to the amino-terminal region of hepatitis B virus preS1 with a W4P mutation. Frontiers in Immunology. 26 February 2021, vol. 12, article no. 637654, pp. 1-12. See abstract; page 2; and figure 1. | 1-14 |
| A | ELIA, Uri et al. Lipid nanoparticle RBD-hFc mRNA vaccine protects hACE2 transgenic mice against a lethal SARS-CoV-2 infection. Nano Letters. 25 May 2021, vol. 21, pp. 4774-4779. See entire document. | 1-14 |
| A | ELIA, Uri et al. Design of SARS-CoV-2 hFc-conjugated receptor-binding domain mRNA vaccine delivered via lipid nanoparticles. ACS Nano. 22 January 2021, vol. 15, pp. 9627-9637. See entire document. | 1-14 |
| A | LEE, Pureum et al. Current status of COVID-19 vaccine development: focusing on antigen design and clinical trials on later stages. Immune Network. February 2021, vol. 21, no. 1, e4, pp. 1-18. See entire document. | 1-14 |
| A | KR 10-2020-0043944 A (LEMONEX INC.) 28 April 2020 (2020-04-28) See entire document. | 1-14 |

☐ Further documents are listed in the continuation of Box C. ☑ See patent family annex.

* Special categories of cited documents:
“A” document defining the general state of the art which is not considered to be of particular relevance
“D” document cited by the applicant in the international application
“E” earlier application or patent but published on or after the international filing date
“L” document which may throw doubts on priority claim(s) or which is cited to establish the publication date of another citation or other special reason (as specified)
“O” document referring to an oral disclosure, use, exhibition or other means
“P” document published prior to the international filing date but later than the priority date claimed
“T” later document published after the international filing date or priority date and not in conflict with the application but cited to understand the principle or theory underlying the invention
“X” document of particular relevance; the claimed invention cannot be considered novel or cannot be considered to involve an inventive step when the document is taken alone
“Y” document of particular relevance; the claimed invention cannot be considered to involve an inventive step when the document is combined with one or more other such documents, such combination being obvious to a person skilled in the art
“&” document member of the same patent family

| Date of the actual completion of the international search | Date of mailing of the international search report |
|---|---|
| **18 August 2022** | **22 August 2022** |

Name and mailing address of the ISA/KR

**Korean Intellectual Property Office**
**Government Complex-Daejeon Building 4, 189 Cheongsa-ro, Seo-gu, Daejeon 35208**

Facsimile No. **+82-42-481-8578**

Authorized officer

Telephone No.

Form PCT/ISA/210 (second sheet) (July 2019)

**INTERNATIONAL SEARCH REPORT**

International application No.
**PCT/KR2022/007697**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/KR2022/007697**

| Patent document cited in search report | Publication date (day/month/year) | Patent family member(s) | Publication date (day/month/year) |
|---|---|---|---|
| KR 10-2020-0043944 A | 28 April 2020 | CN 110475546 A | 19 November 2019 |
| | | EP 3578171 A1 | 11 December 2019 |
| | | JP 2020-506972 A | 05 March 2020 |
| | | JP 2021-143177 A | 24 September 2021 |
| | | JP 6883354 B2 | 09 June 2021 |
| | | KR 10-2018-0091768 A | 16 August 2018 |
| | | KR 10-2019-0095088 A | 14 August 2019 |
| | | KR 10-2152270 B1 | 04 September 2020 |
| | | KR 10-2323574 B1 | 09 November 2021 |
| | | US 11129796 B2 | 28 September 2021 |
| | | US 2020-0009054 A1 | 09 January 2020 |
| | | US 2021-0369614 A1 | 02 December 2021 |
| | | WO 2018-143787 A1 | 09 August 2018 |

Form PCT/ISA/210 (patent family annex) (July 2019)